# EUROPEAN PATENT APPLICATION

(11) **EP 4 170 023 A1**
(43) Date of publication of application: **26.04.2023**
(21) Application number: 21829516.0
(22) Date of filing: 22.06.2021
(51) Int. Cl.: C12N 7/00

(54) **METHOD FOR MASS-PRODUCING VACCINIA VIRUS BY USING SUSPENSION CELLS**

(30) Priority: 22.06.2020 KR 20200075966
(71) Applicant: Kolon Life Science, Inc., Seoul 07793 (KR)
(72) Inventor: KIM, Sung Jin, Seoul 07987 (KR); KIM, Sang Yong, Hwaseong-si Gyeonggi-do 18485 (KR); KIM, Eun Sol, Suwon-si Gyeonggi-do 16675 (KR); KIM, Ka Ul, Seoul 07653 (KR); KIM, Sujeong, Seoul 07062 (KR)
(74) Representative: Ostertag & Partner Patentanwälte mbB
(86) International application number: PCT/KR2021/007835
(87) International publication number: WO 2021/261892

(57) **Abstract**

The present invention relates to a method for mass-producing vaccinia virus using suspended cells. Although methods for producing vaccinia virus using adherent cells in the related art have limitations that are not suitable for mass production of viruses due to the characteristics of adherent cells, the present inventors have developed a technique capable of producing viruses even in a bioreactor using a low appropriate cell number, MOI, culture FBS concentration, and a medium while using suspended cells, and it was also confirmed that the present invention has high virus productivity similar to that in the case of using adherent cells. Accordingly, the technique of producing vaccinia virus using suspended cells according to the present invention enables mass production of vaccinia virus with high productivity. Since it is possible to reduce production costs and time, manpower, and the like using suspended cells, it is expected that the technique will be effectively used in clinical and commercial production fields that require mass production of vaccinia virus.

## Description

### [Technical Field]

The present invention relates to a method for mass-producing vaccinia virus using suspended cells.

### [Background Art]

Vaccinia virus is also called cowpox virus, belongs to the Poxviridae family and is an enveloped DNA virus with a double-stranded linear DNA genome of about 180 kb, which encodes about 250 independent genes. Vaccinia virus has a wide range of hosts such as mammals and birds and is proliferated in various cultured cells to form a white fork which is similar to, but larger than the variola virus. Infection with the vaccinia virus is generally very mild and may induce a rash and fever, but does not cause symptoms in healthy individuals. Since an immune response caused by vaccinia virus infection protects the human body from fatal smallpox infection, vaccinia virus has been used as a live virus vaccine against smallpox.

Vaccinia virus has attracted attention not only as a smallpox vaccine but also as a vaccine delivery system, and has a relatively large linear DNA genome, and thus, can carry various antigenic genes. Further, vaccinia virus has a strong ability to induce immunity, and thus may be used as a vaccine delivery system for a vaccine for treating and preventing an infectious disease, an anticancer vaccine, and the like, which are difficult to develop, and by inserting a foreign gene into an attenuated vaccinia virus, it is possible to produce a recombinant vaccine that is safer and has fewer side effects.

Furthermore, a cancer treatment technique using a vaccinia virus as an oncolytic virus has been recently developed. For example, although an oncolytic vaccinia viral vector, which was developed by SillaJen, Inc., is the only one that has entered clinical phase III, the corresponding clinical trial has been recently suspended by accepting a denial recommendation by the Data Monitoring Committee (DMC), and a clinical trial in combination with immune checkpoint inhibitors is in progress.

Meanwhile, most of the vaccinia viruses, which can be used for various purposes as described above, are produced in adherent cells. SillaJen, Inc. filed an application for a method of producing a vaccinia virus using adherent cells and a roller bottle, HeLa cells, which are uterine cancer cells, were infected with vaccinia virus at an MOI of 0.01 to 0.05 pfu/cell and cultured to confirm a productivity of 50 pfu, and it was concluded that when vaccinia virus is produced using HeLa S3, which is a suspended cell, HeLa S3 is not suitable as a cell line for producing the above virus due to the low productivity of the vaccinia virus (US Patent Publication No. US 2014/0162342). In another patent, a method of producing the NYCBOH strain of vaccinia virus in MRC,-5 which is a human fibroblast, has been filed for application, and another paper reported a method of producing temperature-dependent proteins rather than vaccinia virus using HeLa S3 and a vaccinia virus WR strain.

As described above, in most of the relevant companies of the corresponding techniques and the reported related art, methods of producing vaccinia virus using adherent cells such as Vero, MRC-5, and HeLa is known and used. However, since such existing production methods generate increases in manpower, costs, and time caused by limitations in scale-up due to the characteristics of adherent cells, the existing production methods are not suitable for mass production of the virus.

### [Disclosure]

### [Technical Problem]

Accordingly, the present inventors secured a technique for the production of vaccinia virus using suspended cells, which exhibits a high level of virus productivity, by exploring and evaluating relevant conditions from initial culture using suspended cells to virus infection and production in order to establish a suitable production process capable of mass-producing vaccinia virus.

Accordingly, an object of the present inventors is to provide a method for mass-producing vaccinia virus using suspended cells.

However, technical problems to be solved by the present invention are not limited to the aforementioned problems, and other problems that are not mentioned may be clearly understood by those skilled in the art from the following description.

### [Technical Solution]

In order to achieve the object of the present invention as described above, the present invention provides a method for mass-producing vaccinia virus, the method including the steps of:
(a) initially culturing suspended HeLa S3 or Madin-Darby canine kidney (MDCK) cells;
(b) subculturing the initially cultured cells, seeding the subcultured cells at a density of 5.00E+04 to 1.00E+05 cells/mL, and then infecting the cells with vaccinia virus at a multiplicity of infection (MOI) of 0.01 to 0.1 TCID50/cell and culturing the infected cells; and
(c) harvesting the virus from the cell culture.

As an exemplary embodiment of the present invention, the initial culturing in Step (a) may be culturing the cells until passage 2 to 4.

As another exemplary embodiment of the present invention, the initial culturing in Step (a) may be culturing the cells until passage 2.

As still another exemplary embodiment of the present invention, in Step (a), cells in each passage may be cultured for 3 to 5 days.

As yet another exemplary embodiment of the present invention, in the initial culturing, cells in passage 1 may be seeded at a density of 1.00E+05 to 3.00E+05 cells/mL, and cells in passage 2 may be seeded at a density of 5.00E+04 to 1.00E+05 cells/mL.

As yet another exemplary embodiment of the present invention, the cells may be cultured in a medium supplemented with fetal bovine serum (FBS).

As yet another exemplary embodiment of the present invention, the medium may be a serum-like modified Eagle's medium (SMEM) or a RPMI 1640 medium.

As yet another exemplary embodiment of the present invention, the fetal bovine serum may be added at a concentration of 5% to 10%.

As yet another exemplary embodiment of the present invention, the harvesting in Step (c) may be performed 4 to 6 days after infecting the cells with the virus.

As yet another exemplary embodiment of the present invention, the vaccinia virus may be any one strain selected from the group consisting of Western Reserve (WR), New York vaccinia virus (NYVAC), The New York City Board of Health (Wyeth), LC16m8, Lister, Copenhagen, Tian Tan, USSR, TashKent, Evans, International Health Division-J (IHD-J), International Health Division-White (IHD-W), variants thereof and combinations thereof.

### [Advantageous Effects]

Methods for producing vaccinia virus using adherent cells in the related art have limitations that are not suitable for mass production of viruses due to the characteristics of adherent cells. However, the present inventors developed a technique capable of producing viruses even in a bioreactor using a low appropriate cell number, MOI, culture FBS concentration, and medium while using suspended cells, and it was also confirmed that the present invention has high virus productivity similar to that in the case of using adherent cells. Accordingly, the technique of producing vaccinia virus using suspended cells according to the present invention enables mass production of vaccinia virus with high productivity. Since it is possible to reduce production costs and time, manpower, and the like using suspended cells, it is expected that the technique will be effectively used in clinical and commercial production fields that require mass production of vaccinia virus.

### [Description of Drawings]

FIG. 1A illustrates results obtained by measuring the live cell number daily while seeding and culturing cells at a density of 3.00E+05, 5.00E+05, and 1.00E+06 cells/mL, respectively, in order to select the initial (P+1) culture conditions of suspended HeLa S3 cells.
FIG. 1B illustrates results obtained by measuring cell viability daily while seeding and culturing cells at a density of 3.00E+05, 5.00E+05, and 1.00E+06 cells/mL, respectively, in order to select the initial (P+1) culture conditions of suspended HeLa S3 cells.
FIG. 1C illustrates results obtained by measuring the cell expansion fold daily while seeding and culturing cells at a density of 3.00E+05, 5.00E+05, and 1.00E+06 cells/mL, respectively, in order to select the initial (P+1) culture conditions of suspended HeLa S3 cells.
FIG. 2A illustrates results obtained by measuring the live cell number daily while seeding and culturing cells at a density of 1.00E+05, 3.00E+05, and 5.00E+05 cells/mL, respectively, in order to select the initial (P+1) culture conditions of suspended HeLa S3 cells.
FIG. 2B illustrates results obtained by measuring cell viability daily while seeding and culturing cells at a density of 1.00E+05, 3.00E+05, and 5.00E+05 cells/mL, respectively, in order to select the initial (P+1) culture conditions of suspended HeLa S3 cells.
FIG. 2C illustrates results obtained by measuring the cell expansion fold daily while seeding and culturing cells at a density of 1.00E+05, 3.00E+05, and 5.00E+05 cells/mL, respectively, in order to select the initial (P+1) culture conditions of suspended HeLa S3 cells.
FIG. 3A illustrates results obtained by measuring the live cell number daily while seeding and culturing cells at a density of 5.00E+04, 1.00E+05, and 2.00E+05 cells/mL in Step P+2, respectively, in order to select the initial (P+2) culture conditions of suspended HeLa S3 cells.
FIG. 3B illustrates results obtained by measuring cell viability daily while seeding and culturing cells at a density of 5.00E+04, 1.00E+05, and 2.00E+05 cells/mL in Step P+2, respectively, in order to select the initial (P+2) culture conditions of suspended HeLa S3 cells.
FIG. 3C illustrates results obtained by measuring the cell expansion fold daily while seeding and culturing cells at a density of 5.00E+04, 1.00E+05, and 2.00E+05 cells/mL in Step P+2, respectively, in order to select the initial (P+2) culture conditions of suspended HeLa S3 cells.
FIG. 4A illustrates results obtained by measuring the live cell number daily while seeding and culturing cells at a density of 5.00E+04, 1.00E+05, and 2.00E+05 cells/mL in P+3, respectively, in order to select the initial (P+3) culture conditions of suspended HeLa S3 cells.
FIG. 4B illustrates results obtained by measuring cell viability daily while seeding and culturing cells at a density of 5.00E+04, 1.00E+05, and 2.00E+05 cells/mL in P+3, respectively, in order to select the initial (P+3) culture conditions of suspended HeLa S3 cells.
FIG. 4C illustrates results obtained by measuring the cell expansion fold daily while seeding and culturing cells at a density of 5.00E+04, 1.00E+05, and 2.00E+05 cells/mL in P+3, respectively, in order to select the initial (P+3) culture conditions of suspended HeLa S3 cells.
FIG. 5 illustrates a cell culture process describing cell seeding density and FBS concentration conditions at P+3 in order to determine the FBS concentration during and after infection of suspended HeLa S3 cells with vaccinia virus.
FIGS. 6A to 6H illustrate results obtained by measuring the live cell number (FIGS. 6A to 6C) and cell viability (FIGS. 6D to 6H) after culturing HeLa S3 cells according to the process of FIG. 5 by varying the conditions of cell seeding density and FBS concentration at P+3.
FIG. 7 illustrates the cell culture and virus production process for HeLa S3 cells in order to analyze the cell seeding density at P+3 and virus productivity according to FBS concentration conditions during and after infection with vaccinia virus.
FIG. 8A illustrates results obtained by measuring the cell number after HeLa S3 cell culture and virus production according to the process of FIG. 7 by varying the conditions of cell seeding density and FBS concentration at P+3.
FIG. 8B illustrates results obtained by measuring viability after HeLa S3 cell culture and virus production according to the process of FIG. 7 by varying the conditions of cell seeding density and FBS concentration at P+3.
FIG. 8C illustrates results obtained by measuring virus productivity (TCID50/cell) after HeLa S3 cell culture and virus production according to the process of FIG. 7 by varying the conditions of cell seeding density and FBS concentration at P+3.
FIG. 9 illustrates the cell culture and virus production process (primary experiment) for cell growth and virus productivity analysis according to the vaccinia virus infection MOI, harvest day, and cell seeding density conditions at P+3 for HeLa S3 cells.
FIG. 10A illustrates the results of harvesting samples and measuring the live cell number on each harvest day after performing an experiment according to the process of FIG. 9 by varying the infection MOI when the cell seeding density at P+3 is 1.00E+05.
FIG. 10B illustrates the results of harvesting samples and measuring cell viability on each harvest day after performing an experiment according to the process of FIG. 9 by varying the infection MOI when the cell seeding density at P+3 is 1.00E+05.
FIG. 10C illustrates the results of harvesting samples and measuring the live cell number on each harvest day after performing an experiment according to the process of FIG. 9 by varying the infection MOI when the cell seeding density at P+3 is 5.00E+05 (FIG. 10B).
FIG. 10D illustrates the results of harvesting samples and measuring cell viability on each harvest day after performing an experiment according to the process of FIG. 9 by varying the infection MOI when the cell seeding density at P+3 is 5.00E+05 (FIG. 10B).
FIG. 11A illustrates the results of confirming the total virus production amount of each sample harvested in FIG. 10 in order to analyze virus productivity.
FIG. 11B illustrates the results of confirming the ability of each sample harvested in FIG. 10 to produce viruses per cell (TCID50/cell) in order to analyze virus productivity.
FIG. 12A illustrates the results of confirming the live cell number by performing a secondary experiment for determining the vaccinia virus infection MOI, harvest day, and cell seeding density conditions at P+3 for HeLa S3 cells, in which the experiment was performed by setting the seeding density to 5.00E+04 cells/mL and varying the harvest day conditions, and harvesting samples on each harvest day.
FIG. 12B illustrates the results of confirming viability by performing a secondary experiment for determining the vaccinia virus infection MOI, harvest day, and cell seeding density conditions at P+3 for HeLa S3 cells, in which the experiment was performed by setting the seeding density to 5.00E+04 cells/mL and varying the harvest day conditions, and harvesting samples on each harvest day.
FIG. 12C illustrates the results of confirming the live cell number by performing a secondary experiment for determining the vaccinia virus infection MOI, harvest day, and cell seeding density conditions at P+3 for HeLa S3 cells, in which the experiment was performed by setting the seeding density to 1.00E+05 cells/mL and varying the harvest day conditions, and harvesting samples on each harvest day.
FIG. 12D illustrates the results of confirming viability by performing a secondary experiment for determining the vaccinia virus infection MOI, harvest day, and cell seeding density conditions at P+3 for HeLa S3 cells, in which the experiment was performed by setting the seeding density to 1.00E+05 cells/mL and varying the harvest day conditions, and harvesting samples on each harvest day.
FIG. 13A illustrates the results of confirming the total virus production amount of the samples harvested in FIGS. 12A to 12D in order to analyze virus productivity.
FIG. 13B illustrates the results of confirming the ability of the samples harvested in FIGS. 12A to 12D to produce viruses per cell (TCID50/cell) in order to analyze virus productivity.
FIG. 14A illustrates the results of analyzing the live cell number by performing an experiment under conditions in which the seeding density of cells and the infection MOI were fixed, and then harvesting all the samples on day 3, 4, 5, 6, and 7, respectively, in order to additionally select the day on which cells and the culture solution after virus infection were harvested.
FIG. 14B illustrates the results of analyzing cell viability by performing an experiment under conditions in which the seeding density of cells and the infection MOI were fixed, and then harvesting all the samples on day 3, 4, 5, 6, and 7, respectively, in order to additionally select the day on which cells and the culture solution after virus infection were harvested.
FIG. 15A illustrates the results of confirming the total virus production amount using the samples harvested in FIGS. 14A and 14B.
FIG. 15B illustrates the results of confirming the ability to produce viruses per cell using the samples harvested in FIGS. 14A and 14B.
FIG. 16 illustrates a process for virus production scale-up in which conditions selected for the initial culture conditions of HeLa S3 cells, cell seeding density for virus infection, MOI and harvest day were applied through examples.
FIG. 17A illustrates the results of confirming the cell number and viability before and after scale-up after performing an experiment according to the process of FIG. 16.
FIG. 17B illustrates the results of confirming virus productivity before and after scale-up after performing an experiment according to the process of FIG. 16.
FIG. 18 illustrates an experimental process for selecting a culture medium for HeLa S3 cells.
FIG. 19A illustrates the results of confirming the live cell number after performing an experiment by changing the culture medium for HeLa S3 cells to SMEM, JMEM or RPMI 1640 at P+1 to P+3 according to the process of FIG. 18.
FIG. 19B illustrates the results of confirming cell viability after performing an experiment by changing the culture medium for HeLa S3 cells to SMEM, JMEM or RPMI 1640 at P+1 to P+3 according to the process of FIG. 18.
FIG. 20 illustrates the results of comparatively analyzing virus productivity after performing an experiment by changing the culture medium for HeLa S3 cells at P+1 to P+3 according to the process of FIG. 18.

### [Modes of the Invention]

The present inventors secured a technique for the production of vaccinia virus using suspended cells, which exhibits a high level of virus productivity, by exploring and evaluating relevant conditions from initial culture using suspended cells to virus infection and production in order to establish a production process capable of mass-producing vaccinia virus.

Hereinafter, the present invention will be described in detail.

Accordingly, the present invention provides a method for mass-producing vaccinia virus, the method including: (a) initially culturing suspended HeLa S3 or Madin-Darby canine kidney (MDCK) cells; (b) subculturing the initially cultured cells, seeding the subcultured cells at a density of 5.00E+04 to 1.00E+05 cells/mL, and then infecting the cells with vaccinia virus at a multiplicity of infection (MOI) of 0.01 to 0.1 TCID50/cell and culturing the infected cells; and (c) harvesting the virus from the cell culture.

In the present invention, the "vaccinia virus" includes both the vaccinia virus and a vaccinia virus solution containing the same, unless otherwise specified. The vaccinia virus solution is not particularly limited as long as it contains vaccinia virus, and includes, for example, a culture supernatant after culturing host cells infected with the vaccinia virus and a virus suspension after removing impurities from the culture supernatant.

The vaccinia virus may be any one strain selected from the group consisting of Western Reserve (WR), New York vaccinia virus (NYVAC), The New York City Board of Health (Wyeth), LC16m8, Lister, Copenhagen, Tian Tan, USSR, TashKent, Evans, International Health Division-J (IHD-J), International Health Division-White (IHD-W), variants thereof and combinations thereof, and may be preferably IHD-W, but is not limited thereto.

As used herein, the term "suspended cells" refers to cells that proliferate in a suspended state in a medium without adhering to a culture plate during cell culture, and upon subculture, dilution culture is performed without exchanging the culture medium. Suspended HeLa S3 and MDCK cells that can be used to produce vaccinia virus in the present invention are cells which have the characteristics of adherent cells, but can be adjusted so as to grow in a suspension. HeLa S3 is a clonal derivative of the parental HeLa lineage, which is a human uterine cancer cell, and is a cell line suitable for transfection, and MDCK is a canine kidney-derived cell, and is known in the art as a cell line capable of proliferating various viruses.

In the present invention, an optimal production process that can mass-produce vaccinia virus using suspended cells was researched and established.

In the present invention, Step (a) is a step of initially culturing suspended HeLa S3 or MDCK cells.

The initial culture may be performed by thawing frozen suspended cells, seeding the thawed suspended cells, and culturing the seeded suspended cells until passage 2 to 4, preferably until passage 2 (P+2). For the initial culture, it is preferable to culture cells of each passage for 3 to 5 days after cell seeding, and more preferable to perform subculture after 4 days of culture.

In addition, in the case of initial culture until passage 2 (P+2), it is preferable to seed cells of passage 1 at a density of 1.00E+05 to 3.00E+05 cells/mL and cells of passage 2 at a density of 5.00E+04 to 1.00E+05 cells/mL, and more preferably, optimum cell growth may be induced when cells of passage 1 and cells of passage 2 are seeded at 1.00E+05 cells/mL and 5.00E+04 cells/mL, respectively.

In a specific exemplary embodiment of the present invention, it was confirmed that by culturing cells under various conditions and analyzing the live cell number and cell viability in order to select initial culture conditions before infecting suspended HeLa S3 cells with a virus, specifically the seeding density and culture period of the cells, the above-described conditions were the optimal initial culture conditions for mass-producing vaccinia virus (see Example 1).

The cells may be cultured in a medium supplemented with fetal bovine serum (FBS), and preferably, as a culture medium of cells, a serum-like modified Eagle's medium (SMEM) or a RPMI 1640 medium may be used, and it is more preferable to use RPMI 1640 in terms of culture efficiency of cells and economic feasibility, but the medium is not limited thereto. In this case, the fetal bovine serum may be added at a concentration of 5% to 10%, and may be included at a concentration of preferably 10% during the initial culture of Step (a).

In addition to the conditions selected for the initial culture, the culture temperature, carbon dioxide concentration, and method of subculturing suspended cells are not particularly limited, and the initial culture may be appropriately performed by those skilled in the art according to typical conditions and methods used for the cell culture in the related art.

In the present invention, Step (b) is a step of infecting seeded cells with vaccinia virus after initial culture. For example, in the case of initial culture until passage 2 (P+2), Step (b) is a step of culturing the initially cultured cells to passage 3 (P+3) and seeding the subcultured cells, and then infecting the seeded cells with vaccinia virus.

In Step (b), the seeding density of the cells during infection with the virus is preferably 5.00E+04 to 1.00E+05 cells/mL, and more preferably, when the cells are seeded at 5.00E+04 cells/mL, optimum cell growth and virus production may be induced.

In addition, during infection with the virus, the multiplicity of infection (MOI) is preferably 0.01 to 0.1 TCID50/cell, and more preferably, when cells are infected at a MOI of 0.01, the highest cell growth and virus productivity may be induced.

As used herein, the term "multiplicity of infection (MOI)" refers to the ratio of an agent (for example, virus) to an infection target (for example, host cell). It refers to the ratio of the number of viral particles to the number of target cells present in a limited space.

Furthermore, in Step (b), as a medium used to culture virus-infected suspended cells, a SMEM or RPMI 1640 medium supplemented with 5% to 10% FBS may be used, and more specifically, it is preferable to use a medium supplemented with 5% FBS.

The present inventors confirmed through examples that the above-described conditions in Step (b) are optimum mass production conditions.

That is, in another specific exemplary embodiment of the present invention, in order to determine the concentration of FBS added during infection of suspended HeLa S3 cells with a virus and culture after the infection, cells were seeded at different densities and cultured at a concentration of 0, 2, 5 and 10% FBS, respectively, and then the cell growth and productivity of the vaccinia virus were comparatively analyzed, and the optimum cell seeding density and FBS concentration for virus infection were selected as described above (see Example 2).

In still another exemplary embodiment of the present invention, experiments were performed to select viral infection MOI, cell and medium harvesting period, and cell seeding density conditions for virus infection in suspended HeLa S3 cells. Specifically, when the cells were seeded at different densities and MOIs and harvesting periods were varied, cell growth and vaccinia virus productivity were compared to select the optimum conditions as described above (see Examples 3-1 to 3-3).

In the present invention, the productivity of the vaccinia virus was evaluated by the total virus production amount and the ability to produce viruses per cell (TCID50/cell). The ability to produce viruses per cell is synonymous with 'titer', which is often used in the art as a unit indicating a virus infection titer. Since viruses cannot be seen using an optical microscope, their density (number/volume) cannot be measured under a microscope like biological cells. Therefore, in the case of viruses, an infectious titer using infectivity with respect to host cells is used as a unit, and replaces the amount or concentration thereof. For example, when a virus suspension diluted to an appropriate ratio is added to a monolayer of host cells, the number of viruses is detected as plaques, and the infectious titer may be measured as plaque forming unit (pfu)/mL. Alternatively, the infectious titer may be measured by diluting a liquid containing the virus and setting a concentration at which the percentage of generating a positive infection for host cells becomes 50% as a 50% tissue culture infectious dose (TCID50)/mL. In the present example, the productivity of the vaccinia virus, that is, the infectious titer, was measured as TCID50/mL, but the present invention is not limited thereto.

In addition to the conditions selected above, the method of culturing suspended cells and infecting cells with a virus is not particularly limited, and those skilled in the art can appropriately apply and implement methods typically used in the art.

In the present invention, Step (c) is a step of harvesting the virus from the cell culture in order to obtain the virus produced through Step (b).

In order to harvest the virus, it is preferable to harvest both the cultured cells and the culture. The virus may be harvested 4 to 6 days after infection with the virus, preferably 5 days after infection with the virus.

From the sample harvested through the step, those skilled in the art may obtain a finally produced virus by a method used in the art and analyze the productivity.

In yet another exemplary embodiment of the present invention, a virus production scale-up experiment was performed on a 1.8 L scale in order to examine whether the production conditions of the vaccinia virus of the present invention can be applied to an actual mass production process of the virus, and then the live cell number, cell viability and virus productivity were analyzed, respectively. As a result, it was confirmed that in two scales (30 mL and 1800 mL), both cell number and viability were shown to be similar, and the virus productivity was also similar. Through this, it was confirmed that the production process and established conditions for the vaccinia virus using suspended cells according to the present invention can be applied to the mass production scale (see Examples 3 and 4).

In yet another exemplary embodiment of the present invention, an additional experiment for selecting a culture medium for suspended HeLa S3 cells was performed in order to further enhance virus productivity or secure a production cost reduction effect in the selected virus production process. As a result, it was confirmed that RPMI 1640 also exhibited similar cell growth and virus production effects compared to SMEM used in the steps, and based on these results, it was determined that it would be appropriate to use the RPMI 1640 medium, which is easier to supply and can reduce costs (see Example 4).

The vaccinia virus finally produced through the production process of the present invention can be variously used in basic research and clinical fields such as vaccines, oncolytic viruses for the treatment of cancer, and viral vectors as delivery systems.

Hereinafter, preferred examples for helping with understanding of the present invention will be suggested. However, the following examples are provided only so that the present invention may be more easily understood, and the content of the present invention is not limited by the following examples.

### [Examples]

### Example 1. Selection of culture conditions for suspended HeLa S3 cells

In order to establish the culture conditions for the suspended HeLa S3 cells, which is a cell line for producing vaccinia virus, the present inventors performed an experiment for selecting initial culture conditions from P+1 to P+3 after cell thawing.

### 1-1. Selection of P+1 culture conditions

First, in order to select the culture conditions for P+1, the cells were thawed and seeded under the conditions shown in the following Table 1, and then the live cell number, cell viability and cell expansion fold were measured daily while culturing the cells.

**[Table 1]**

| | |
|---|---|
| **Cell line** | HeLa S3 |
| **Culture media** | SMEM (10% FBS, 1% AA) |
| **Culture scale** | 30mL in 125mL EMF |
| **RPM** | 110 rpm |
| **Incubator conditions** | 37°C 5% CO₂ |
| **Cell seeding density** | 3.00E+05, 5.00E+05, 1.00E+06 cells/mL |

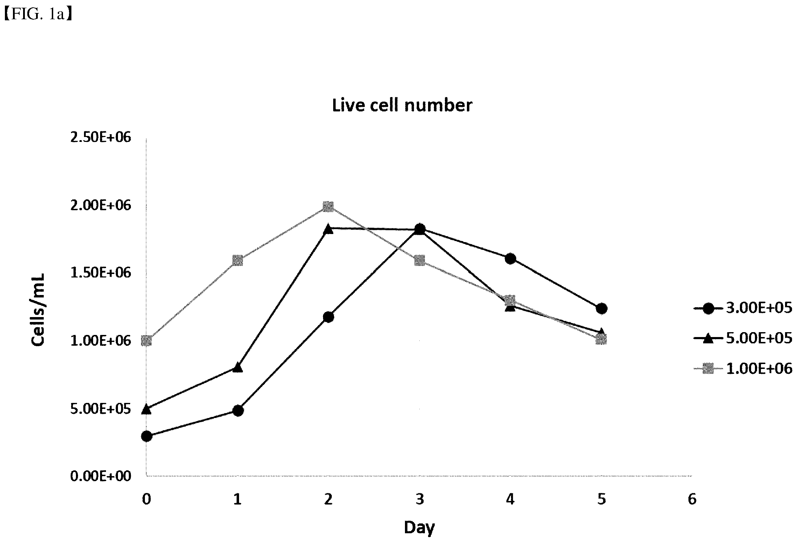

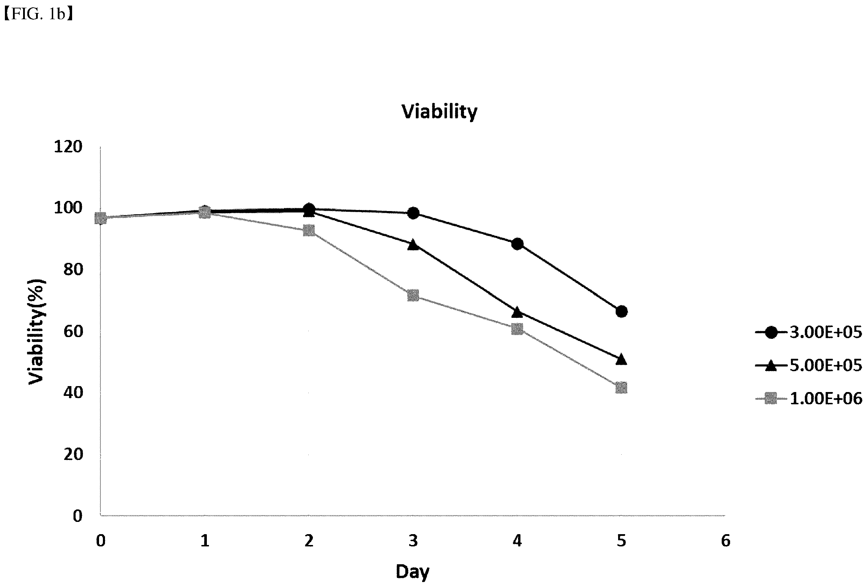

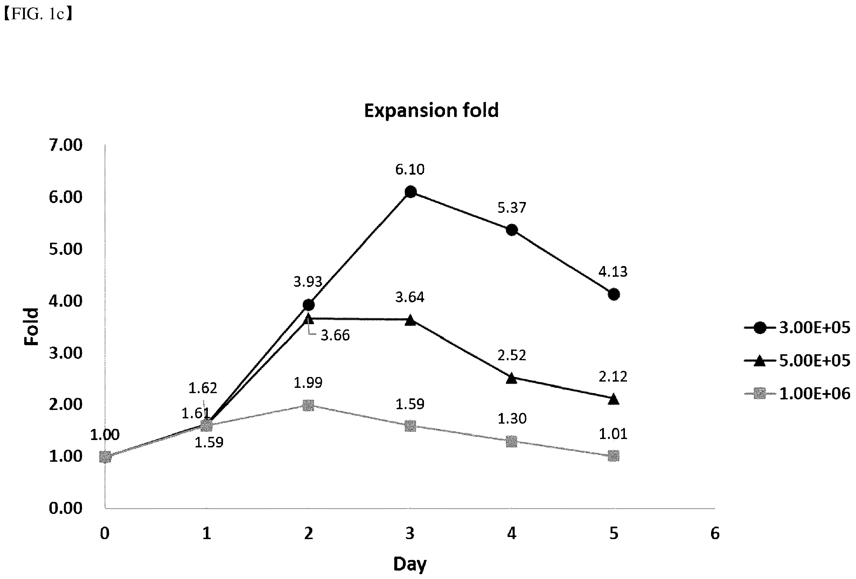

As a result of culturing the cells under the above conditions and performing the experiment, as illustrated in FIGS. 1A to 1C, it was found that the number of cells that maximally grew without replacing the medium was about 2.00E+06 cells/mL, and the higher the density of the cells, a high cell number could be obtained in a faster time. However, as a result of confirming the expansion fold of the cells, it was confirmed that the cells were cultured at the highest efficiency when the cells were seeded at the lowest density of 3.00E+05 cells/mL. Therefore, it could be seen that during cell seeding, the culture efficiency is better when the density of cells is low than when the density of cells is high.

Based on the above results, cell growth was comparatively analyzed by varying the seeding density of cells to 1.00E+05, 3.00E+05, and 5.00E+05 as shown in the following Table 2 using 3.00E+05 cells/mL as a standard in the subsequent experiments.

**[Table 2]**

| | |
|---|---|
| **Cell line** | HeLa S3 |
| **Culture medium** | SMEM (10% FBS, 1% AA) |
| **Culture scale** | 30mL in 125mL EMF |
| **RPM** | 110 rpm |
| **Incubator conditions** | 37°C 5% CO₂ |
| **Cell seeding density** | 1.00E+05, 3.00E+05, 5.00E+05 cells/mL |

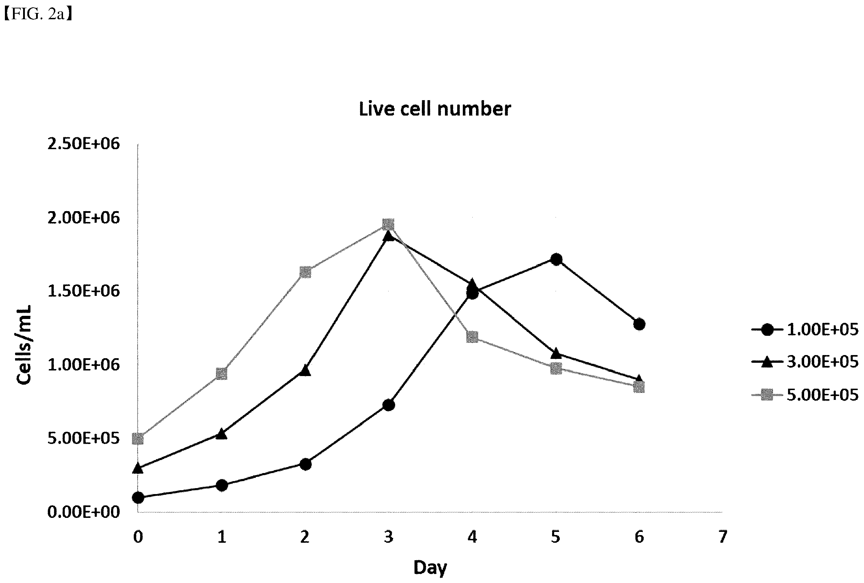

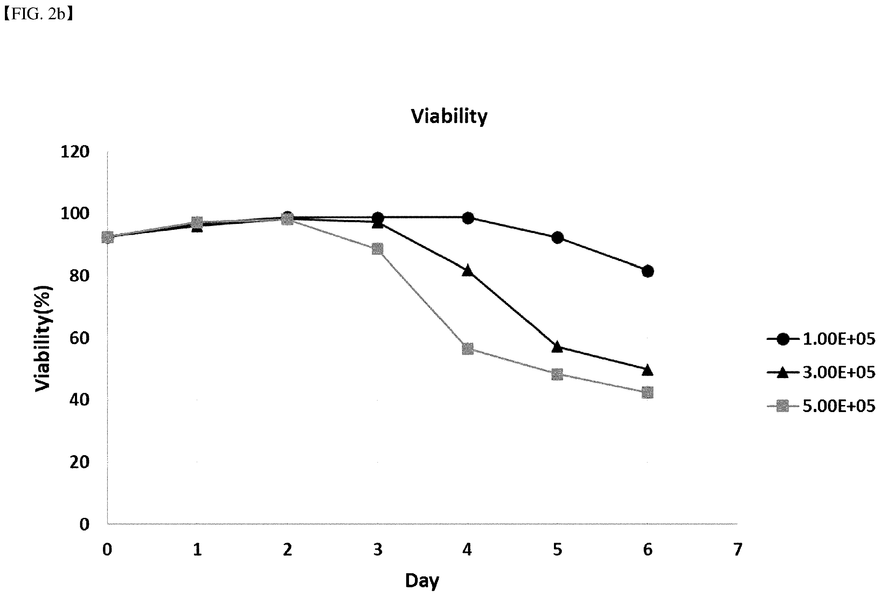

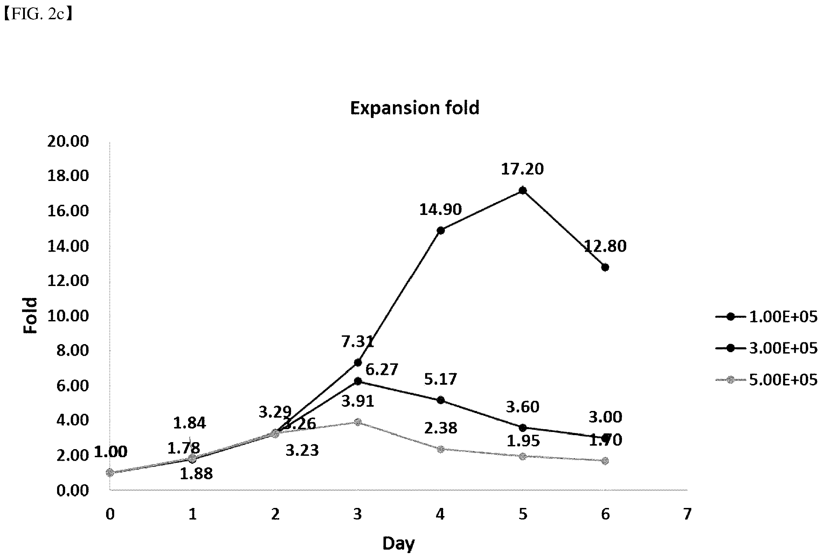

As a result of culturing cells under the conditions in Table 2, as can be seen in FIGS. 2A to 2C, it was confirmed that the maximum number of cells during the culture period was shown to be similar for all three cell density conditions, but viability was shown to be the highest when the cells were seeded at the lowest density of 1.00E+05. Furthermore, under the other two density conditions, the graph peaked and dropped immediately, making it difficult to set a subculture cycle, whereas in the case of 1.00E+05, as the period between days 4 and 5 is shown to be the stationary phase, it was determined that it is appropriate to subculture cells on day 4 of culture.

Accordingly, during the P+1 culture, the conditions of seeding the cells seeded at 1.00E+05 cells/mL and subculturing the cells after 4 days were set.

### 1-2. Selection of P+2 culture conditions

Next, in order to select culture conditions for P+2, the cells were cultured according to the conditions shown in the following Table 3, and cell culture efficiency according to the cell seeding density conditions was comparatively analyzed. In this case, it was observed that the initial high cell density tends to rather result in low culture efficiency through the process of setting the culture conditions for P+1, and in the present experiment, the cell seeding density was further lowered to set the conditions and culture the cells.

**[Table 3]**

| | |
|---|---|
| **Cell line** | HeLa S3 |
| **Culture medium** | SMEM (10% FBS, 1% AA) |
| **Culture scale** | 30mL in 125mL EMF |
| **RPM** | 110 rpm |
| **Incubator conditions** | 37°C 5% CO₂ |
| **Cell seeding density** | 5.00E+04, 1.00E+05, 2.00E+05 cells/mL |

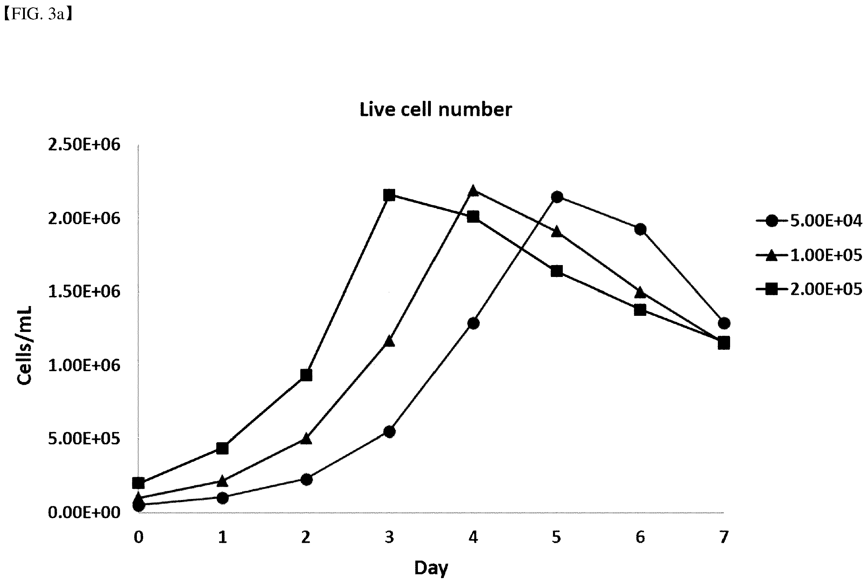

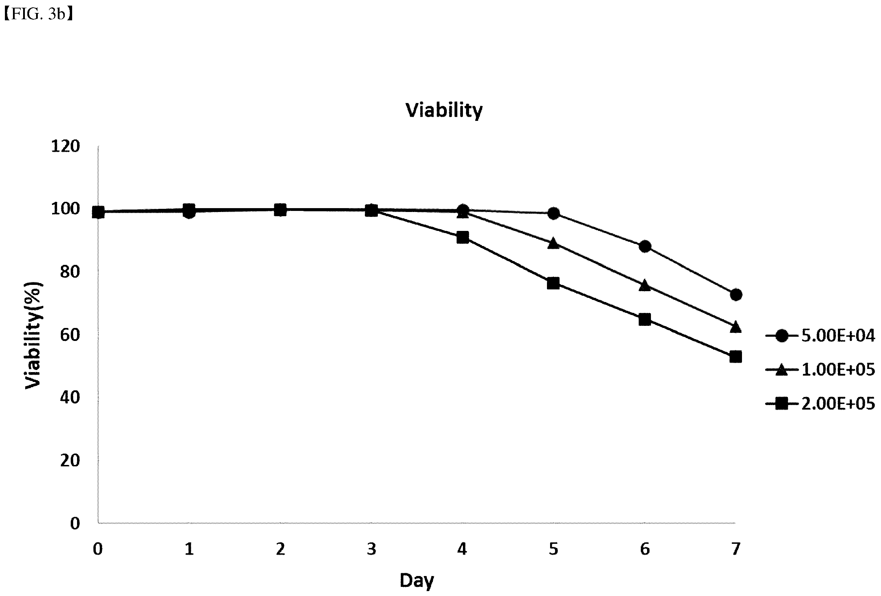

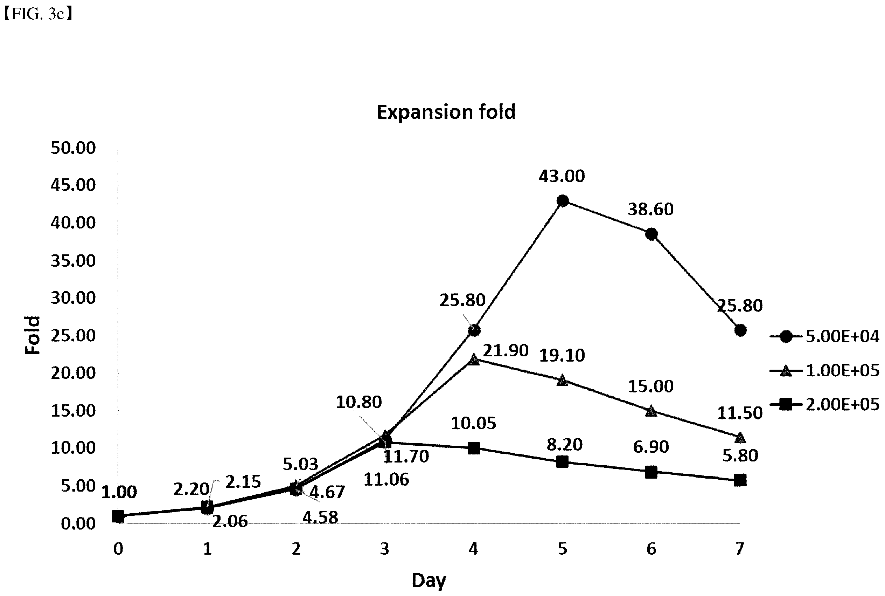

As a result of the experiment, as illustrated in FIG. 3A to FIG. 3C, it was confirmed that the time to reach the maximum cell number differed depending on the seeding density of the cells, but the final cell number was shown to be about 2.00E+06 cells/mL or more in all cases, and the cell efficiency is the best at the lowest density of 5.00E+04 cells/mL from the observation that the cell viability and expansion fold were shown to be the highest at the lowest density. When it is considered that the cells are subcultured in the log phase in selecting the P+2 culture conditions, it was determined that it is suitable to seed the cells at 5.00E+04 cells/mL and subculture the cells on day 4 when the expansion fold was also high while viability is maintained. The above conditions have an advantage in that culture efficiency is so high that the culture scale can be scaled up to about 750 mL at P+3 in the future and convenience is high because the culture cycle is the same as in P+1.

### 1-3. Selection of P+3 culture conditions

Furthermore, in order to select the conditions of P+3, cells were cultured under the P+1 and P+2 conditions selected through Examples 1-1 and 1-2, and then the culture of P+3 was performed under the conditions in the following Table 4.

**[Table 4]**

| | |
|---|---|
| **Cell line** | HeLa S3 |
| **Culture medium** | SMEM (10% FBS, 1% AA) |
| **Culture scale** | 30mL in 125mL EMF |
| **RPM** | 110 rpm |
| **Incubator conditions** | 37°C 5% CO₂ |
| **Cell seeding density** | 5.00E+04, 1.00E+05, 2.00E+05 cells/mL |

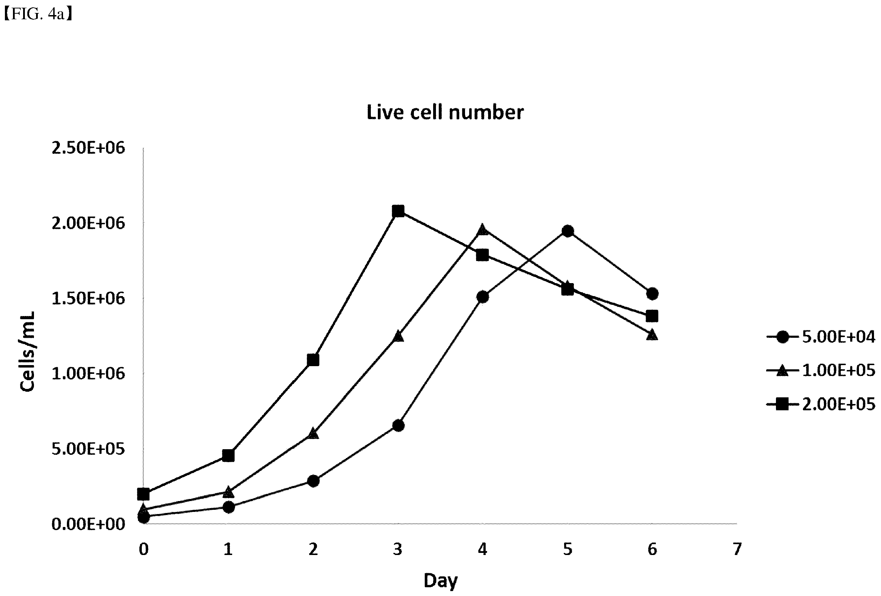

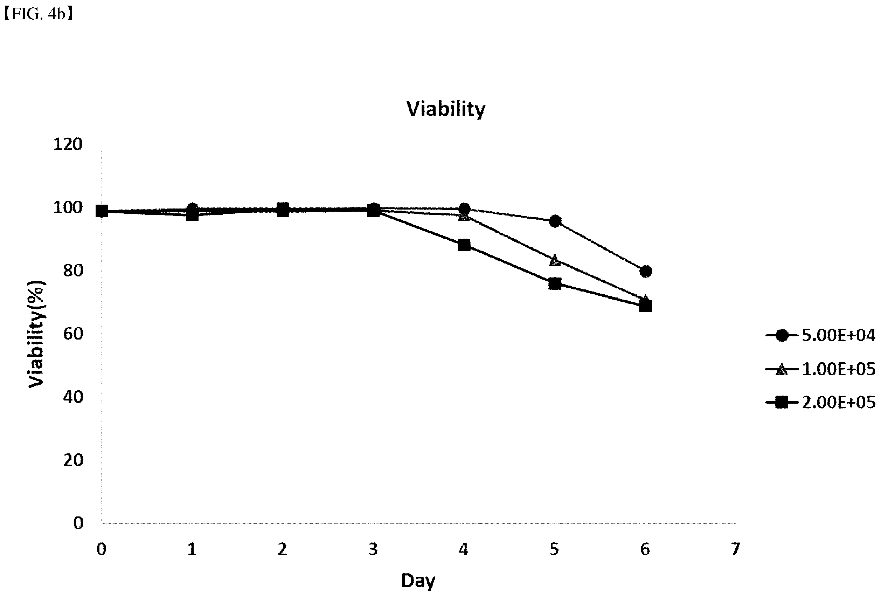

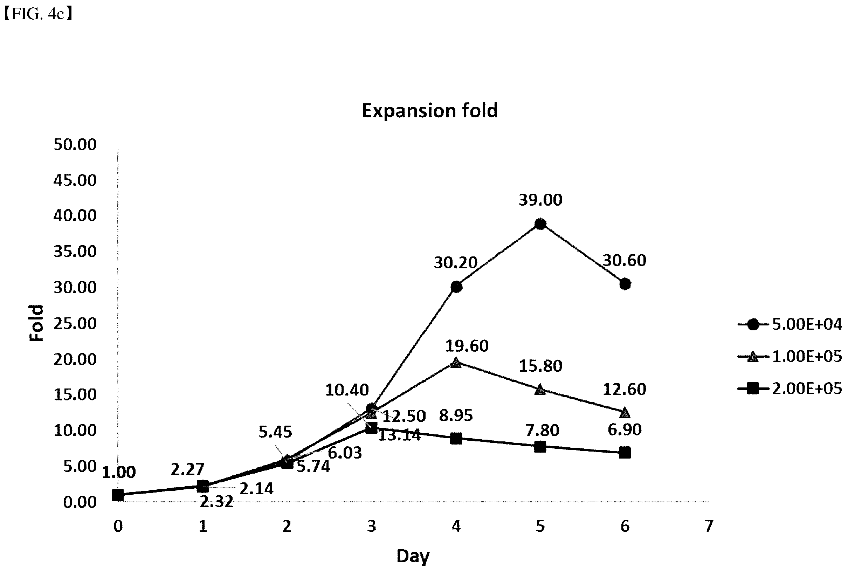

As a result of the culture, as can be seen in FIGS. 4A to 4C, it was confirmed that a growth curve similar to that of P+2 was observed, and the cell culture efficiency was shown to be the highest at 5.00E+05 cells/mL which is a low seeding density. In addition, in consideration of the culture efficiency and cell viability in the log phase state, it was determined that it is appropriate to perform a subculture on day 4 after the cells were cultured in the same manner as in P+2.

Accordingly, the initial culture conditions until P+1, P+2 and P+3 selected in the examples are summarized in the following Table 5, and HeLa S3 cells were cultured under the above conditions during an experiment for selecting the production conditions of vaccinia virus in the future.

**[Table 5]**

| | |
|---|---|
| **Cell line** | HeLa S3 |
| **Culture medium** | SMEM (10% FBS, 1% AA) |
| **Culture scale** | 30mL in 125mL EMF |
| **RPM** | 110 rpm |
| **Incubator conditions** | 37°C 5% CO₂ |
| **P+1 cell seeding density and subcutlure cycle** | 1.00E+05 cells/mL, Day 4 |
| **P+2 cell seeding density and subcutlure cycle** | 5.00E+04 cells/mL, Day 4 |
| **P+3 cell seeding density and subcutlure cycle** | 5.00E+04 cells/mL, Day 4 |

### Example 2. Selection of FBS concentration during culture of suspended HeLa S3 cells

The present inventors conducted the following experiments to select an FBS concentration capable of exhibiting the optimum cell growth and vaccinia virus productivity in the culture of suspended HeLa S3 cells.

### 2-1. Observation of cell growth according to FBS concentration

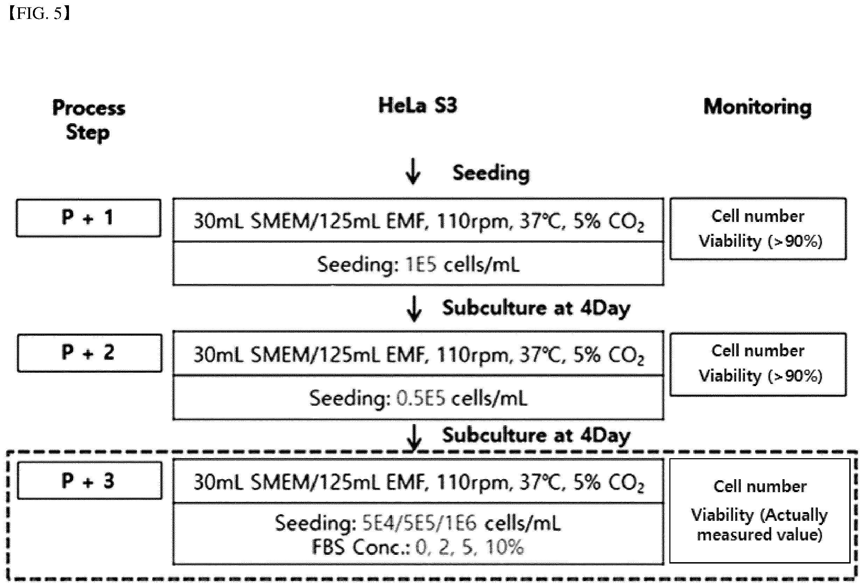

First, in order to observe the cell growth of HeLa S3 according to the FBS concentration in the culture medium, cells were cultured under the conditions selected through Example 1 as in the following FIG. 5, and then the cell seeding density at P+3 was set at 5.00E+04, 5.00E+05, and 1.00E+06 cells/mL, and the growth curve of cells was analyzed while performing the culture by varying the FBS concentration under four conditions (0, 2, 5, and 10%) in each case.

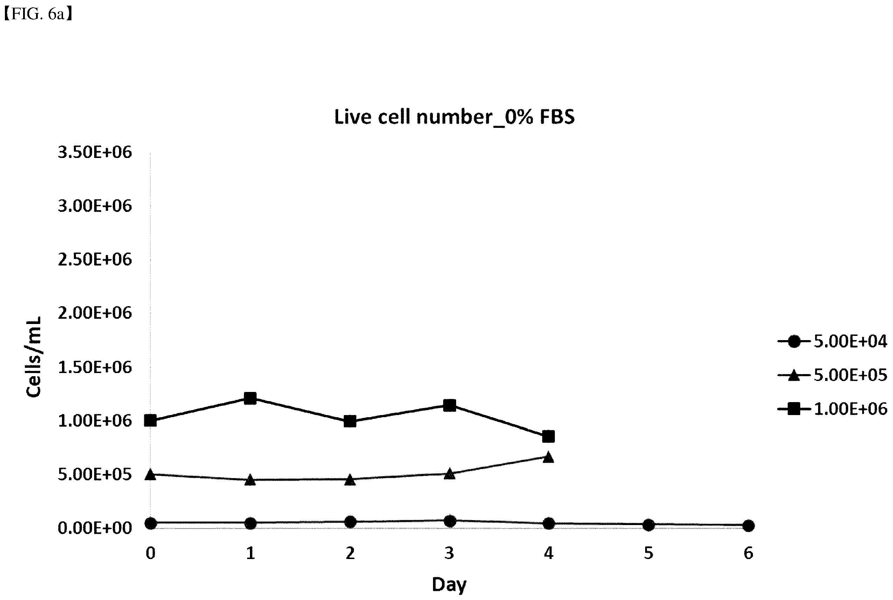

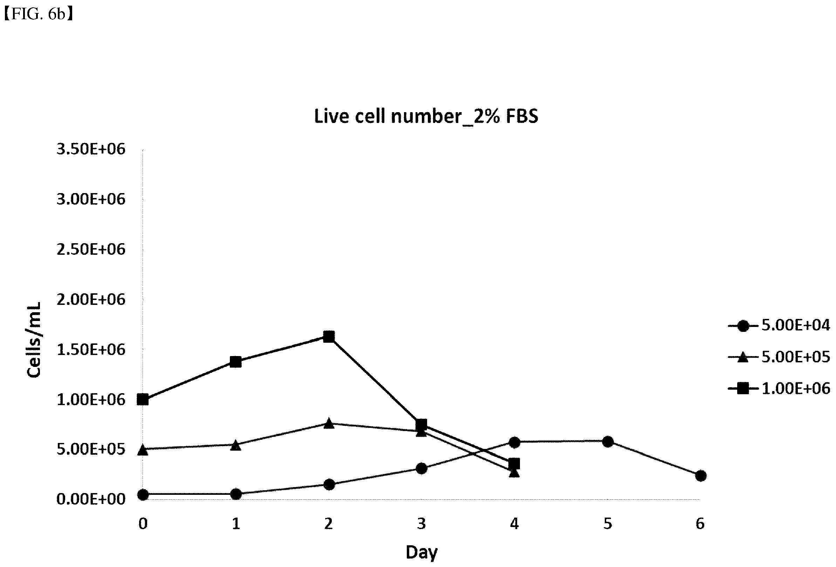

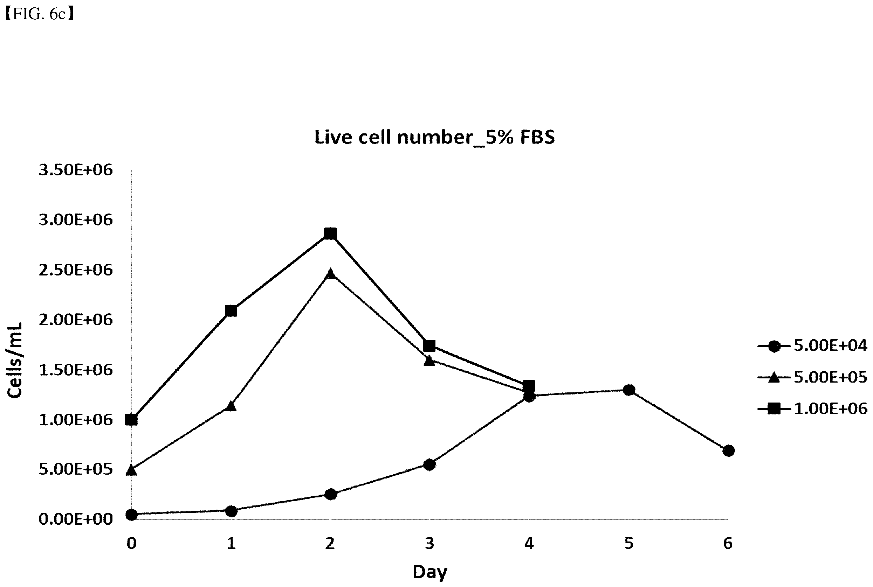

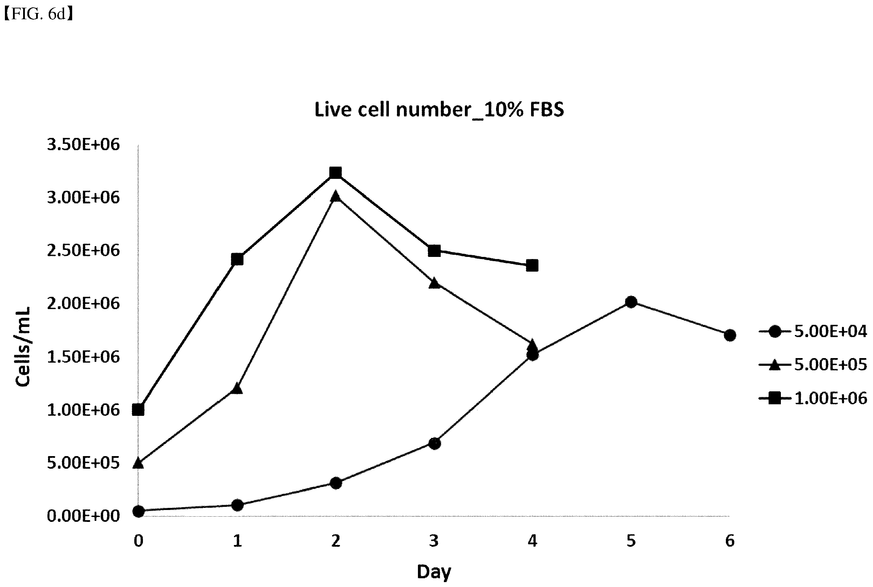

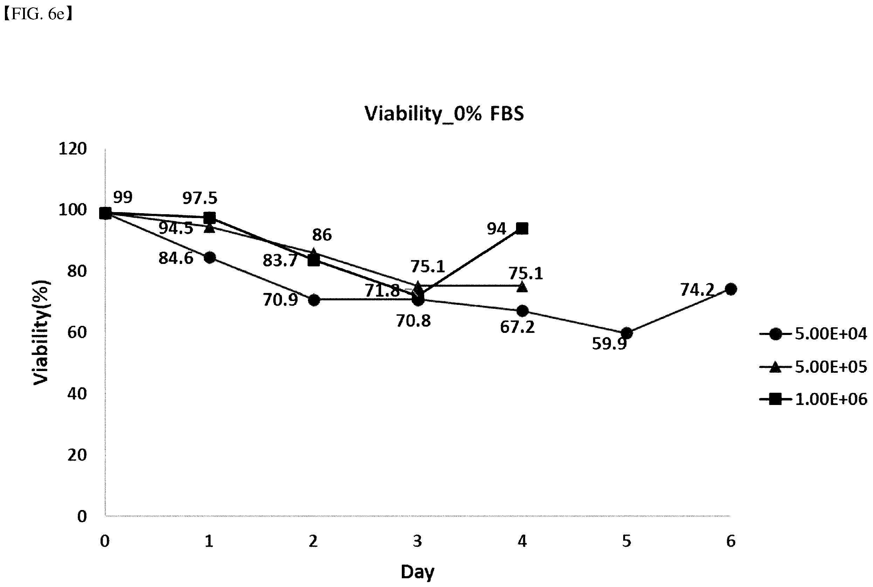

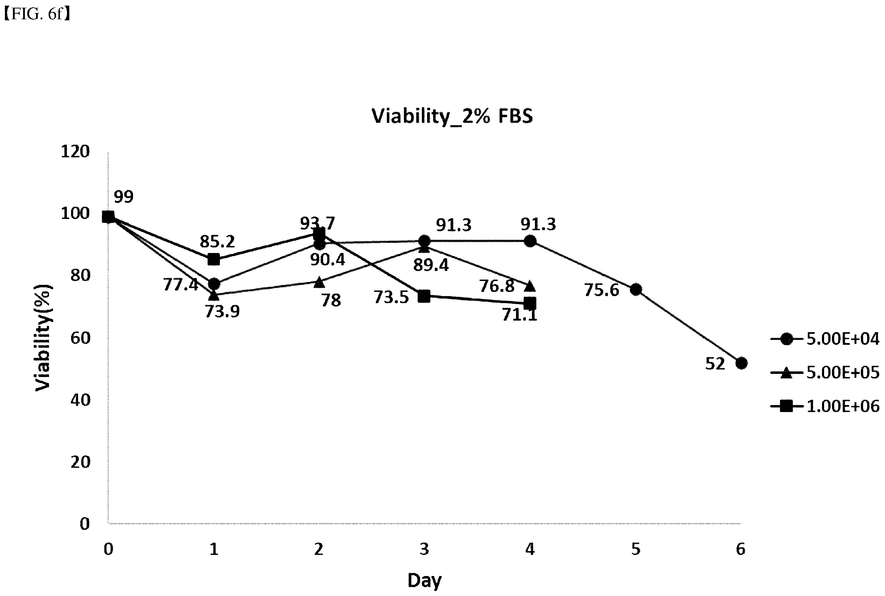

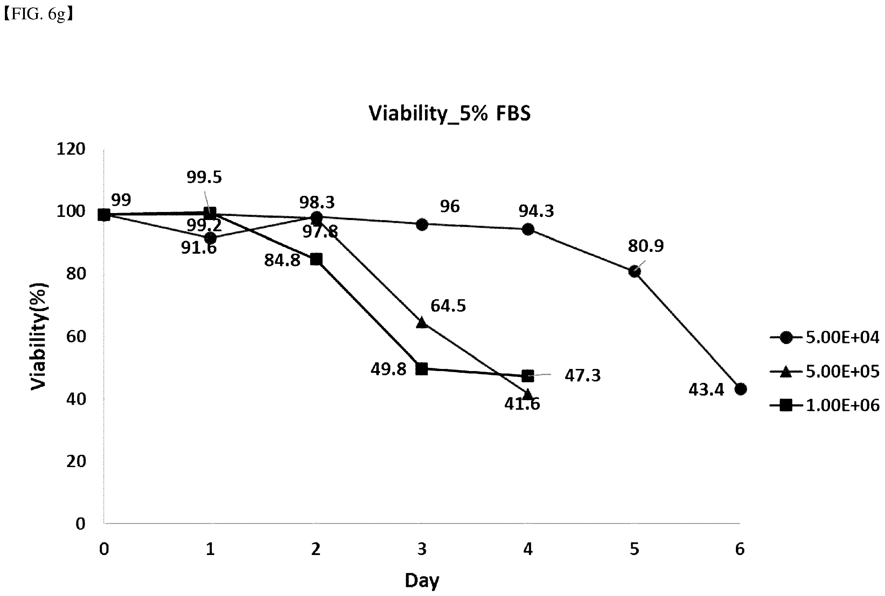

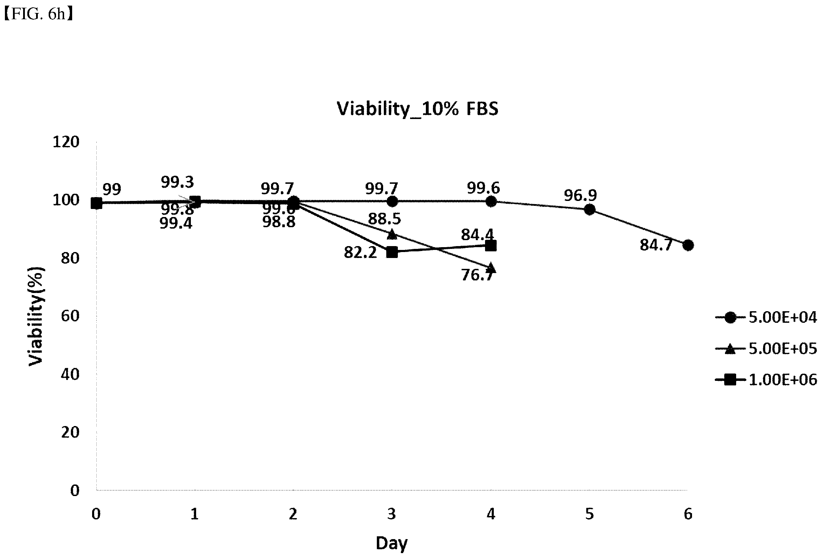

As a result of respectively analyzing the live cell number and cell viability while performing P+3 culture, it was found that the cells did not grow and viability remained low as expected under the condition of 0% FBS, as illustrated in FIGS. 6A to 6H. Although a slight increase in cell number was observed under the conditions of 2% FBS, no significant growth efficiency was observed, and except for the case of 5.00E+04 cells/mL which is the lowest cell density, a decrease in cell viability was observed from the initial phase of culture. In the case of 5% FBS, growth curves similar to those of 10% FBS, which is the positive control, appeared at all cell densities until day 2 of culture, and similar viability was observed for cells at low densities. Through the above results, it was expected that virus productivity would also be low because the state of cells was not good under the condition of 0 to 2% FBS during virus infection and cell culture for the production of vaccinia. However, in order to confirm substantial virus productivity depending on the FBS concentration, a virus productivity verification experiment was performed under the same FBS conditions as described above.

### 2-2.Confirmation of productivity of vaccinia virus (VACV) according to FBS concentration and selection of conditions

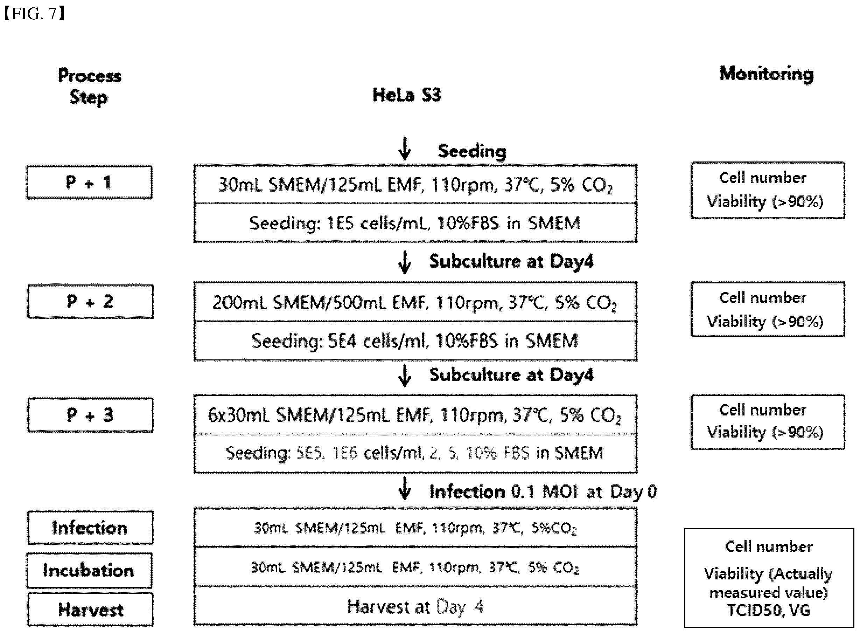

Next, in order to confirm the productivity of vaccinia virus in suspended HeLa S3 cells depending on the FBS concentration, an experiment was according to according to the process of FIG. 7. In this case, cells were infected with vaccinia virus at a cell seeding density of 5.00E+05 or 1.00E+06 cells/mL, an FBS concentration of 2, 5 or 10%, and an MOI of 0.1 TCID50/cell. Subsequently, the cells were cultured for 4 days, both the cells and culture medium were harvested, and virus productivity was evaluated under each condition.

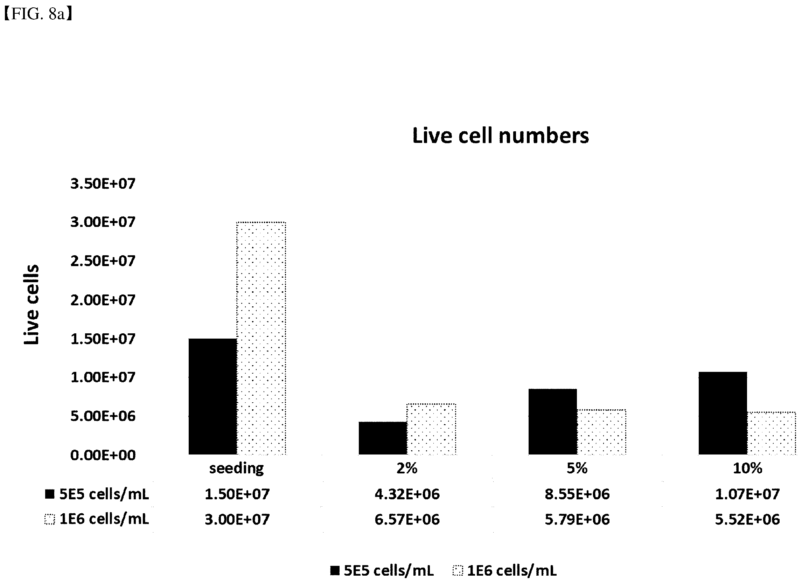

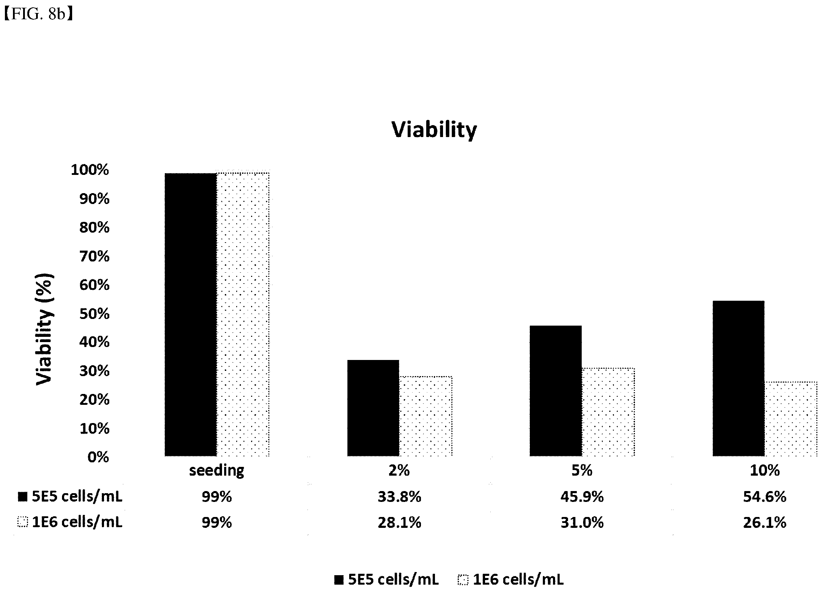

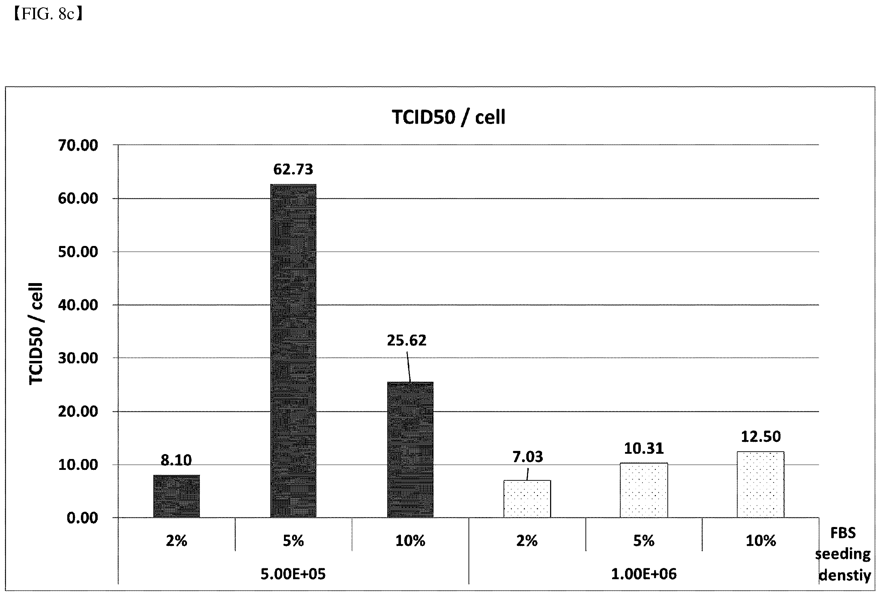

As a result of analyzing the live cell number and cell viability depending on the FBS concentration at each of the two cell density conditions, as illustrated in FIGS. 8A to 8C, it was found that a decrease in cell number and viability was observed as cells were cultured and viruses were produced, and at 5.00E+05 cells/mL, the cell number and viability were also increased slightly as the concentration of FBS was increased. In contrast, in the case of 1.00E+06 cells/mL, the cell number and viability were shown to be similar as the FBS concentration was increased. Through these results, it could be expected that at 5.00E+05 cells/mL, there would be a difference in virus productivity depending on the FBS concentration. Actually, as a result of evaluating the productivity of the vaccinia virus through TCID50 capable of confirming the ability to produce viruses per cell, it was found that there was a large difference in virus productivity depending on each FBS condition at 5.00E+05 cells/mL, and it was confirmed that the productivity was highest under the conditions of 5% FBS. Accordingly, in order to produce vaccinia virus at a high titer, the FBS concentration was finally selected to be 5% during and after cell infection.

### Example 3. Selection of virus infection and harvest conditions in suspended HeLa S3 cells

### 3-1. Primary experiment for selection of virus infection MOI, harvest day and cell seeding density

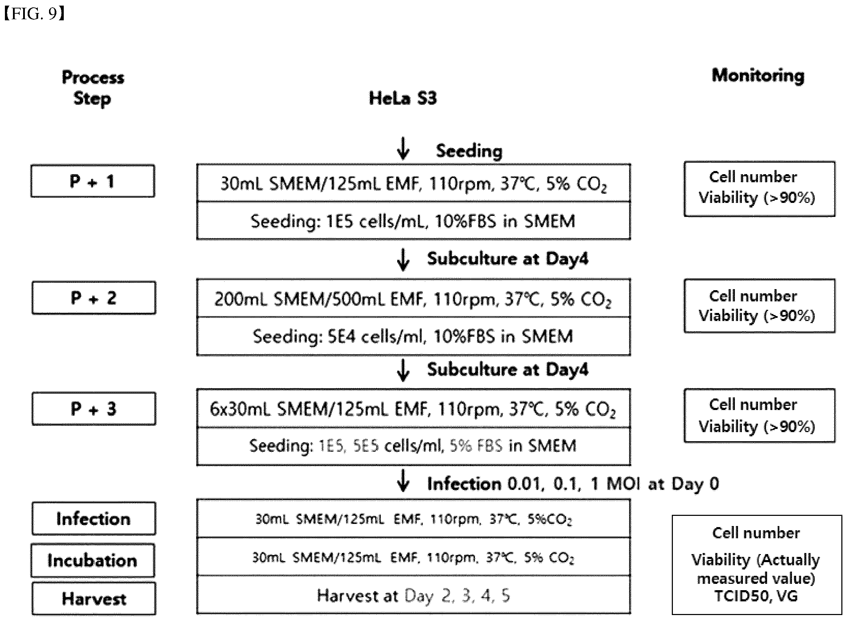

An experiment for selecting the multiplicity of Infection (MOI), harvest day and cell seeding density conditions was performed in order to further improve virus productivity while using the initial culture process of the suspended HeLa S3 cells selected through the experiments in Examples 1 and 2 and the FBS concentration conditions during infection and production of the vaccinia virus. The experiment was performed according to the process and conditions described in FIG. 9, and specifically, the virus infection MOI was set to the conditions of 0.01, 0.1, and 1 TCID50/cell, and on days 2, 3, 4, and 5 after virus infection, virus productivity was evaluated by harvesting both the cells and culture medium, respectively. In addition, the seeding density of cells for virus infection was set to two conditions of 1.00E+05 cells/mL and 5.00E+05 cells/mL.

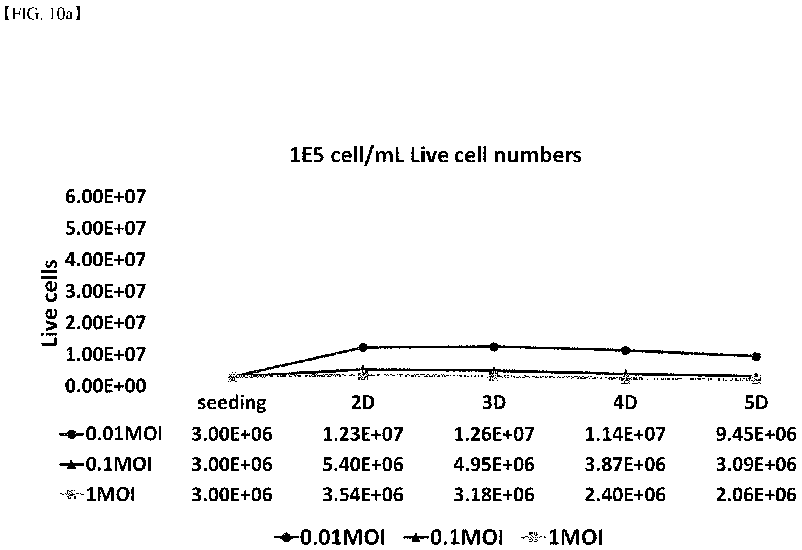

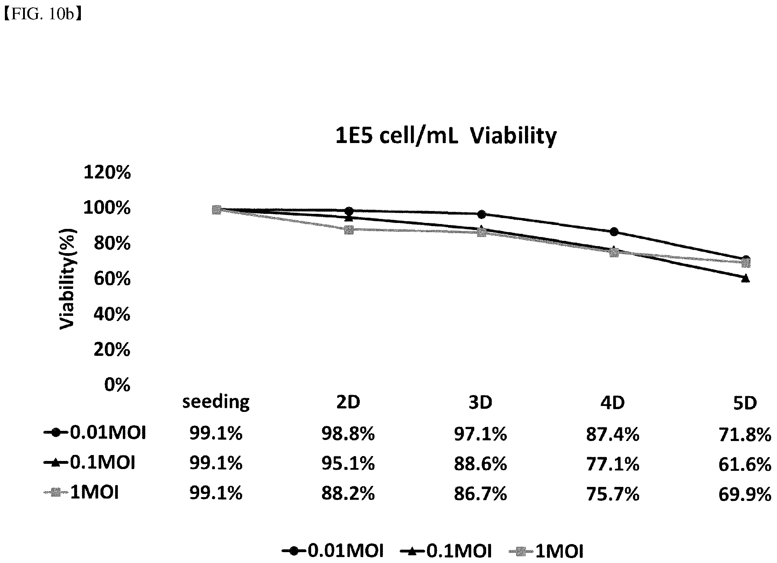

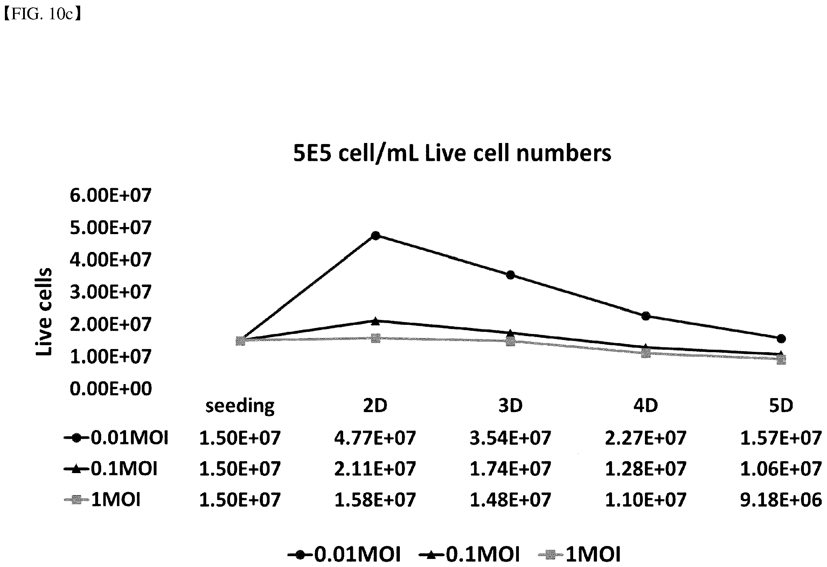

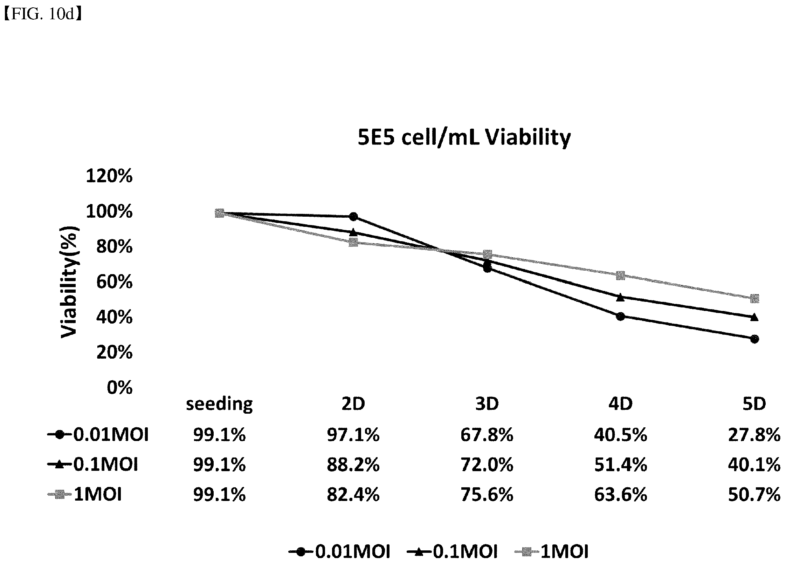

First, as a result of analyzing the live cell number and cell viability according to the MOI and harvest day, respectively, under the two cell density conditions, as illustrated in FIGS. 10A to 10D, at the lowest MOI of 0.01 under both density conditions, it was found that the cell number was increased and then either maintained or decreased, and it was observed that cell viability was decreased as the viral infection concentration and incubation period were increased. Through this pattern, it was possible to predict that viruses were subsequently being produced through virus infection.

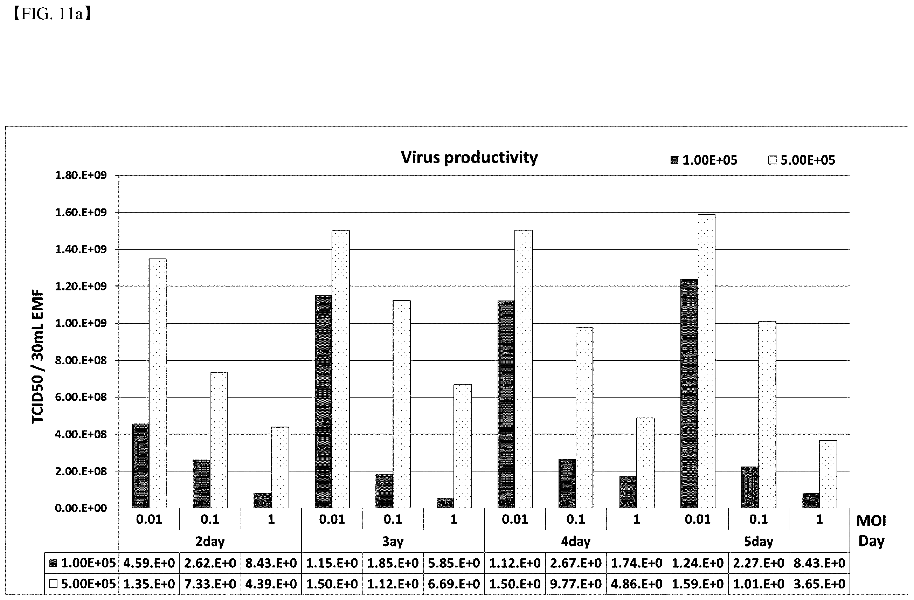

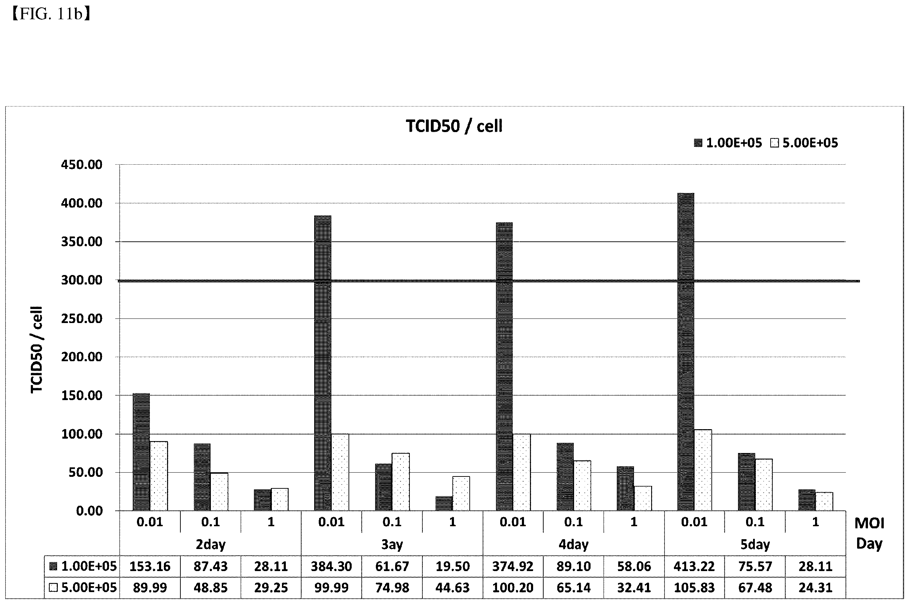

Furthermore, virus productivity was analyzed by performing a TCID50 assay on samples harvested on days 2 to 5 after the start of culture. As a result, as illustrated in FIG. 11A, the total virus productivity was shown to be highest under the conditions where cells were seeded at 5.00E+05 cells/mL and infected at a MOI of 0.01 and harvested after 3, 4, and 5 days. However, as can be seen in FIG. 11B, it was confirmed that the actual ability to produce viruses per cell was the highest when cells were seeded at a density of 1.00E+05 cells/mL, then infected with a virus at MOI of 0.01, and harvested after days 3, 4, and 5. In addition, considering that the vaccinia virus productivity when using an adherent HeLa cell is 300 TCID50/cell or more as an internal standard, it could be confirmed that even when HeLa S3 cells, which are suspended cells, were used under the above conditions according to the present invention, 300 TCID50/cell or more of virus was produced. 5.00E+05 cells/mL uses 5-fold more cells than 1.00E5 cells/mL, but there was no significant difference in the total production amount and the production efficiency was shown to be very low, so the cells were primarily seeded at a density of 1.00E+05 cells/mL, infected with the virus at an MOI of 0.01, and then harvested on days 3 to 5 to select conditions. Furthermore, referring to FIGS. 11A and 11B, it could be observed that the lower the density and MOI of the seeded cells, the higher the virus productivity, so in the subsequent secondary experiment, it was attempted to perform the experiment by preferentially further lowering the cell density.

### 3-2, Secondary experiment for selecting virus infection MOI, harvest day and cell seeding density

Based on the results of Example 3-1, a secondary experiment for selecting the vaccinia virus infection MOI, the harvest day, and the cell seeding density was performed. The overall process was the same as in FIG. 9 performed in Example 3-1, provided that at P+3, the cell seeding density was adjusted to 5.00E+04 and 1.00E+05 cells/mL and in FIGS. 11A and 11B, when the samples were harvested on day 2 after infection, both cells and medium solutions were harvested on days 3, 4, and 5 after infection, respectively, except for day 2 under the harvesting conditions, because the productivities in all the cases was shown to be low.

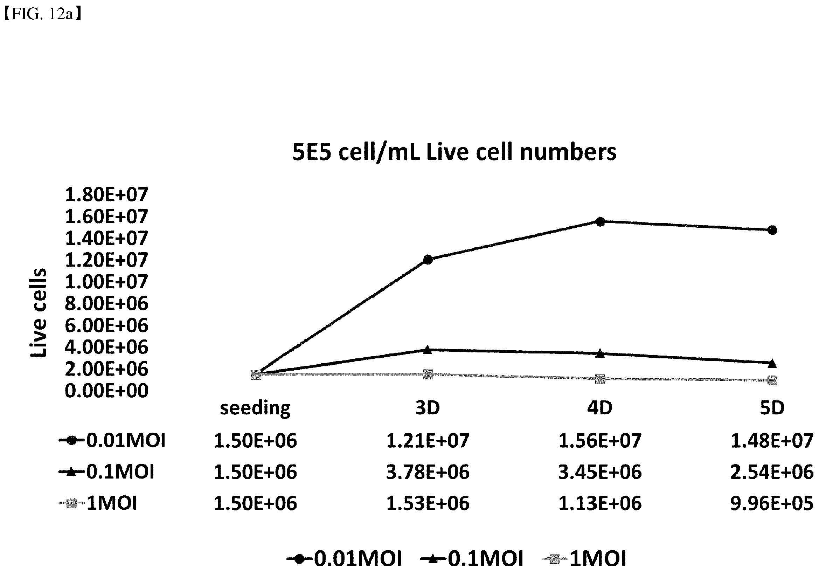

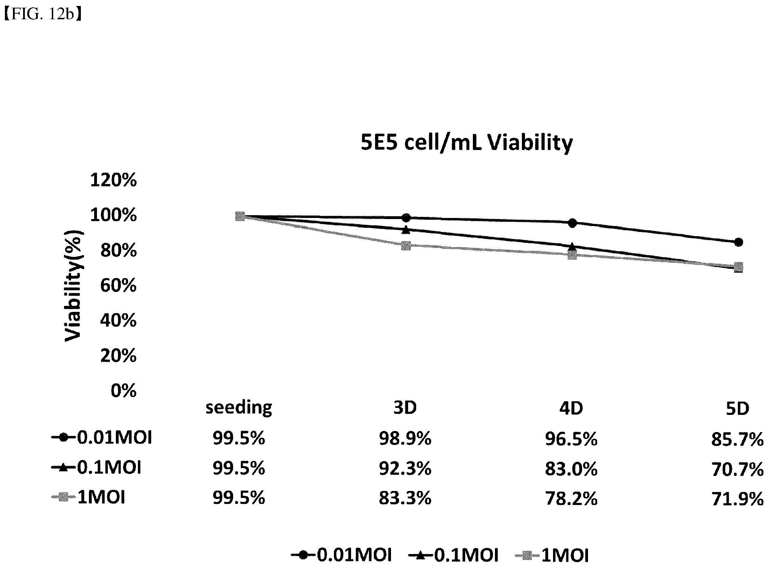

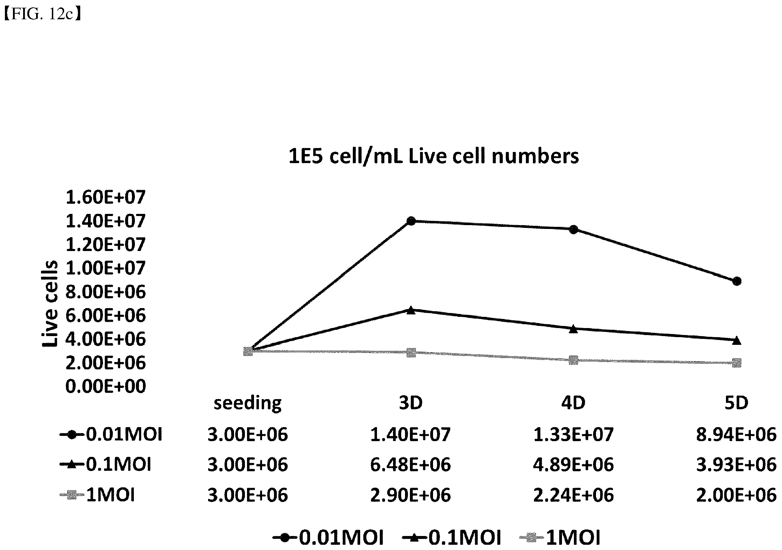

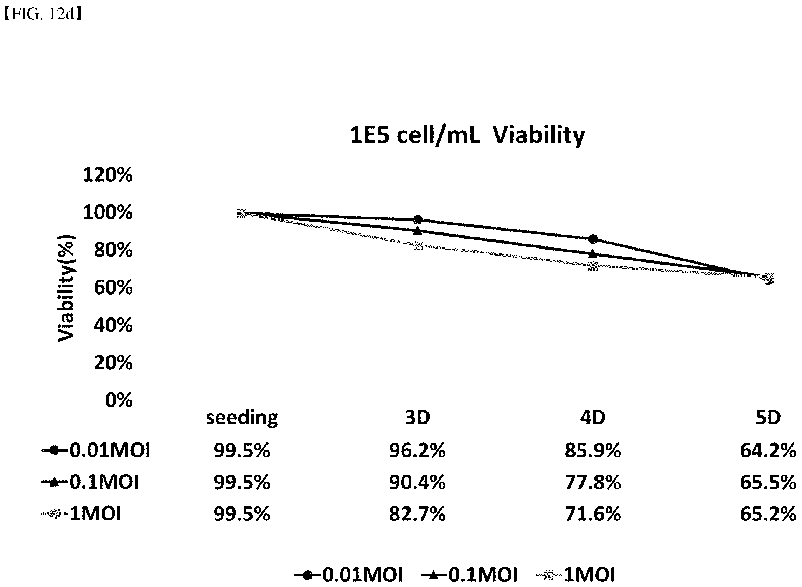

First, as a result of analyzing the live cell number and cell viability depending on the MOI and harvest date under the above two cell density conditions, as illustrated in FIGS. 12A to 12D, it was observed that the cell number was increased and viability was also maintained until day 4 of culture. It was confirmed that at a higher concentration of 1.00E+05 cells/mL, the cell number was increased and viability was maintained until day 3 of culture.

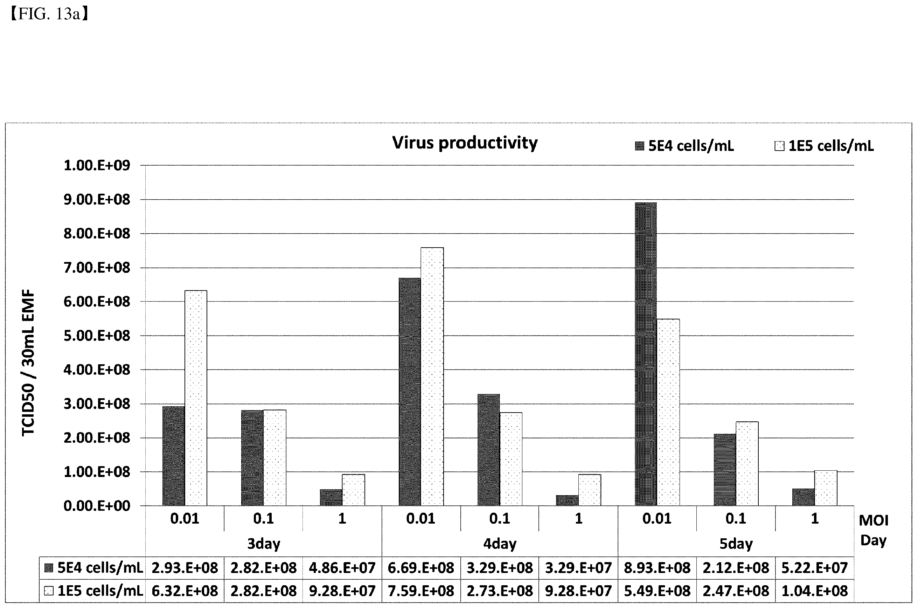

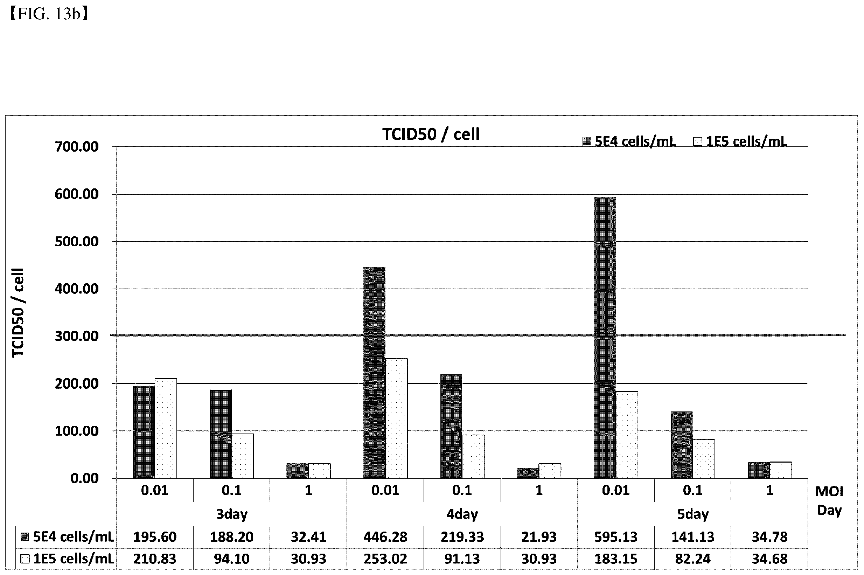

Furthermore, as a result of analyzing virus productivity by infecting the cells with a virus and performing a TCID50 assay on each sample harvested after days 3 to 5 of culture, as illustrated in FIG. 13A, it was confirmed that the virus productivity was higher at a low cell density (5.00E+04 cells/mL) as expected, and as can be seen in FIG. 13B, it was confirmed that viruses were produced at 300 TCID50/cell or more, which is the productivity of the vaccinia virus in an adherent HeLa cell, employed as an internal standard. In particular, the infection condition of MOI of 0.01 showed a pattern in which the productivity continued to increase without declining as the harvest day increased from day 3 to day 5. Based on the above results, the cell seeding density was selected to be 5.00E+04 cells/mL, which is lower than that of the primary experiment in Example 3-1, and conditions, under which the cells were infected with the virus at an MOI of 0.01 TCID50/cell and both the cells and the culture medium were harvested after 5 days, were selected.

### 3-3. Experiment of selecting additional harvest day

Since a pattern in which the productivity is increased as the harvest day is extended is shown in FIG. 13B, the present inventors performed an experiment for verifying whether the productivity of the vaccinia virus is increased by prolonging the harvest day. The overall experimental process was performed similar to that in FIG. 9, which was performed in Example 3-1, provided that at P+2, the SMEM medium was adjusted to 80 mL, and based on the above experimental results, experiments were performed by fixing the cell seeding density for virus infection at 5.00E+04 cells/mL and the MOI at 0.01 TCID50/cell, and only setting the harvest day at days 3, 4, 5, 6, and 7.

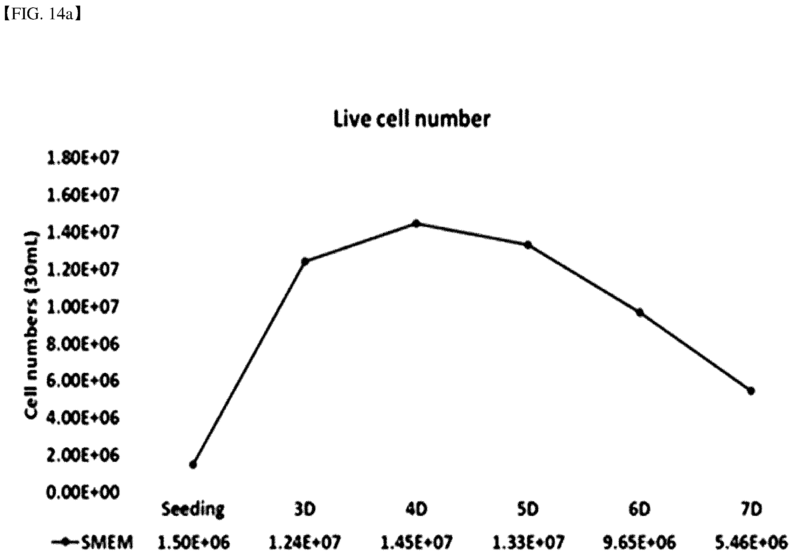

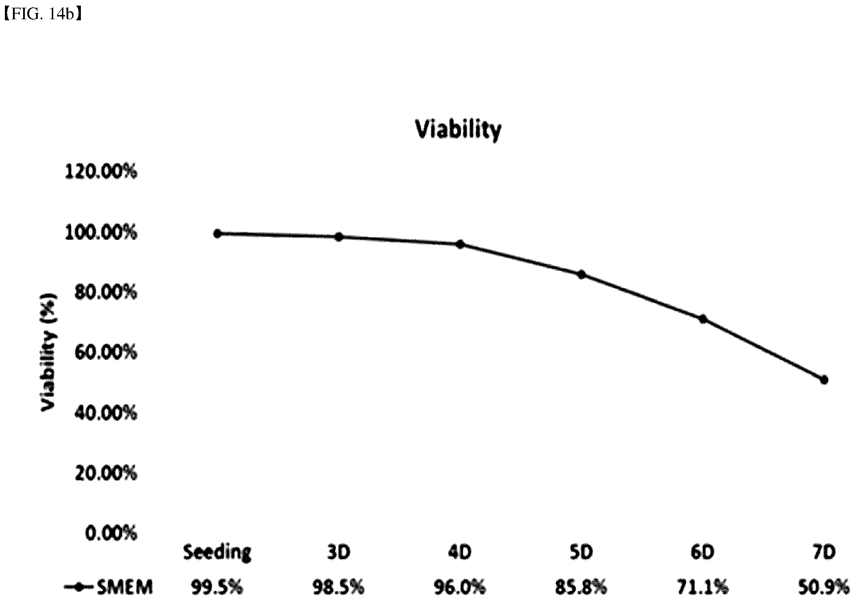

First, as a result of analyzing the cell number and viability, as illustrated in FIGS. 14A and 14B, it was found that the cell number increased until days 3 to 5 after virus infection and then decreased thereafter, and it was confirmed that viability decreased sharply from day 4.

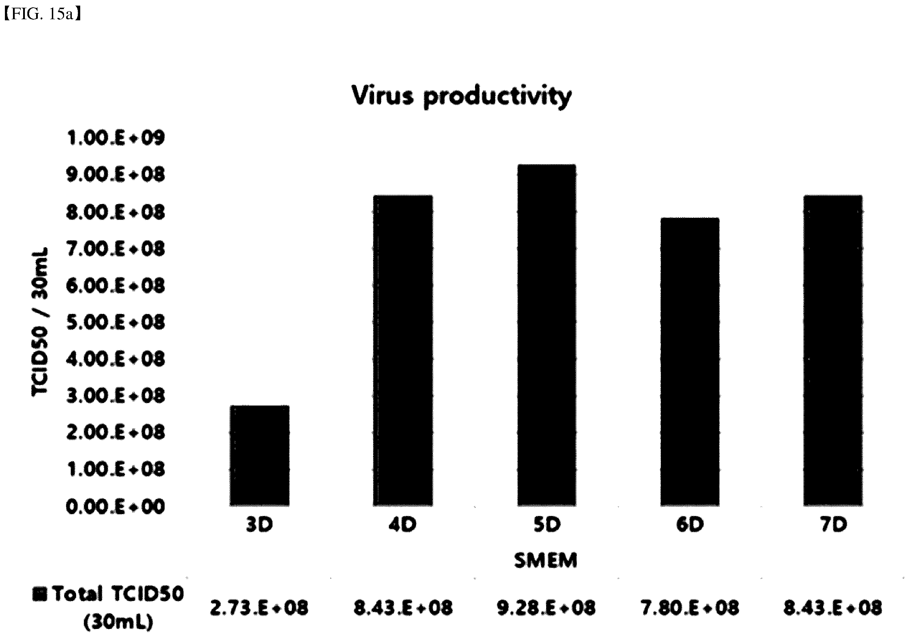

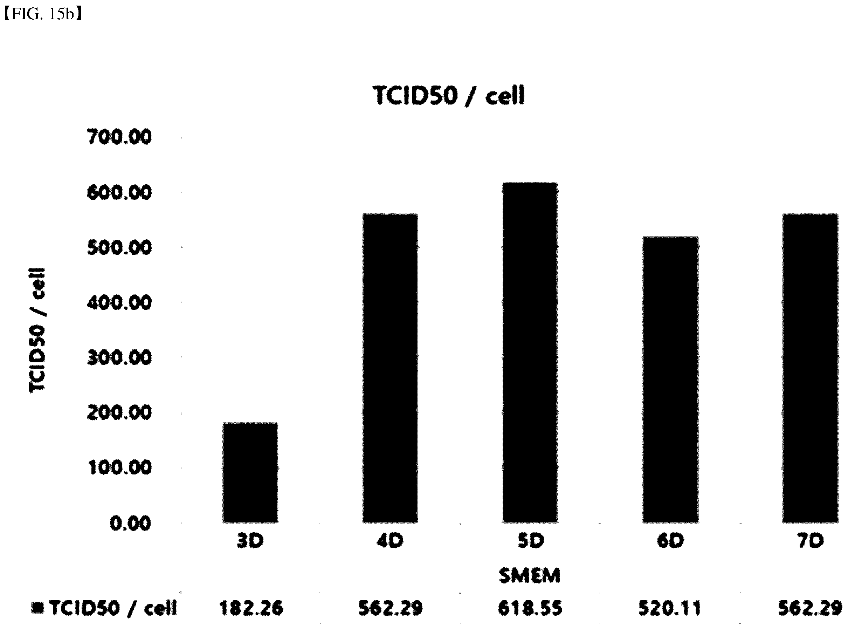

Further, as a result of analyzing the productivity of the vaccinia virus according to the harvest day, as can be seen in FIGS. 15A and 15B, from day 5, it was found that virus production was not increased any more while the cell growth is maintained. According to such results, it was finally selected to infect the cells with the virus and harvest the virus after 5 days.

### 3-4. Scale-up of vaccinia virus production

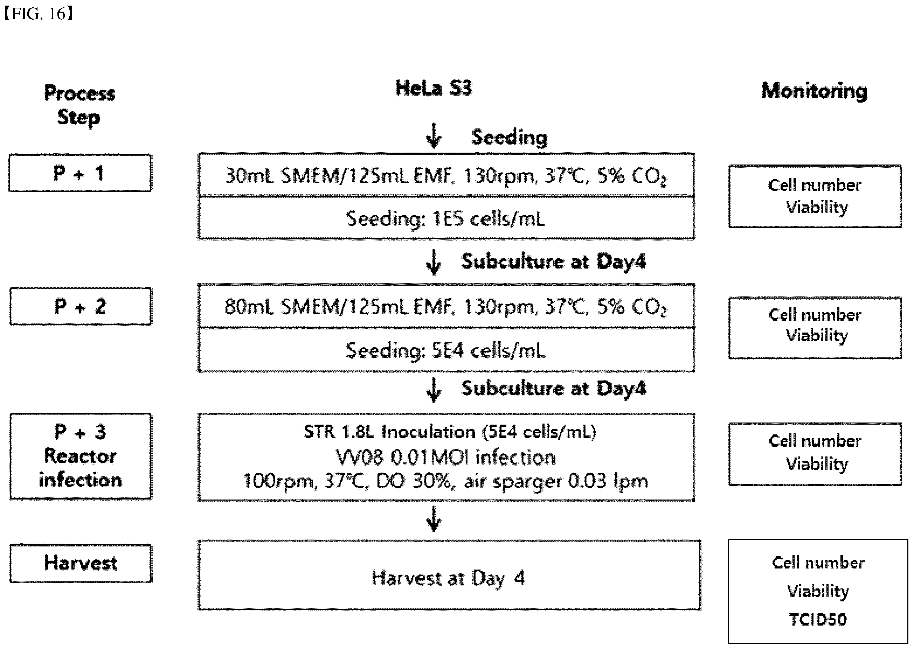

Since conditions for the production of the vaccinia virus, that is, initial cell culture conditions, virus seeding density for infection, MOI and harvest day conditions were selected through the results of the above examples, a virus production scale-up experiment was performed to produce the vaccinia virus on a scale of 1.8 L in a stirred tank reactor (STR). Specifically, the experiment was performed according to the process in FIG. 16, and the productivity of the finally analyzed virus was compared with the results of the 30 mL EMF scale in the above example.

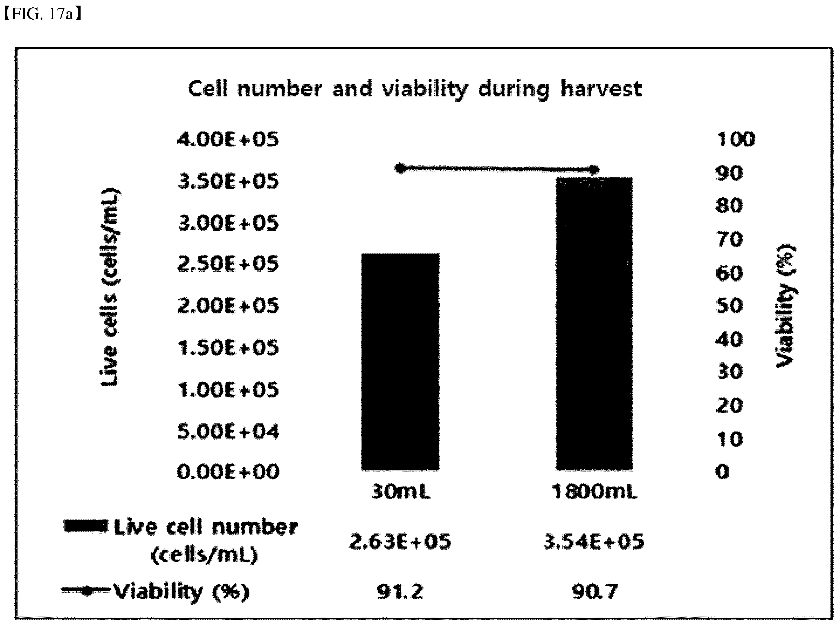

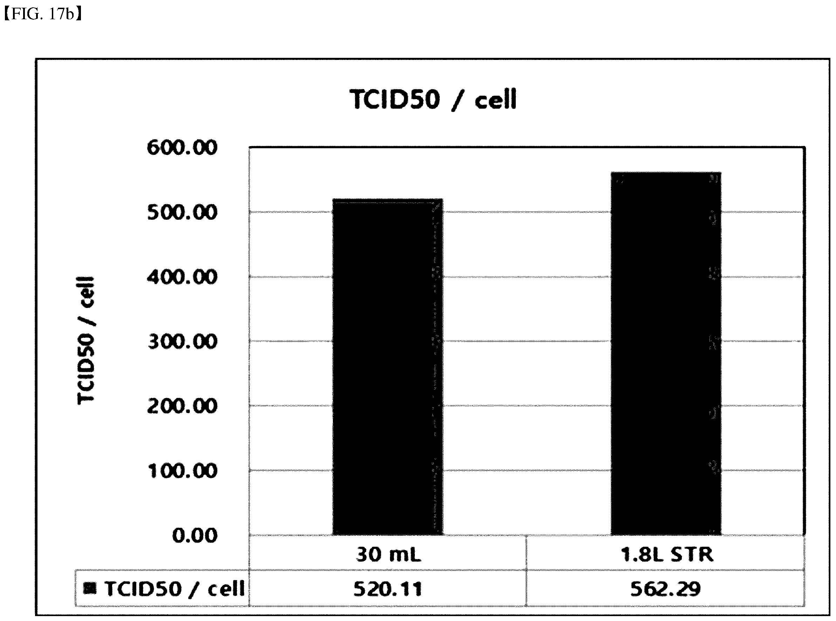

Specifically, as a result of analyzing each of the live cell number, cell viability and virus productivity by infecting cells with the virus and harvesting all samples after 4 days, as illustrated in FIG. 17A, it was confirmed that the cell number and viability were observed to be similar at the two scales (30 mL, 1800 mL), and as can be seen in FIG. 17B, it was confirmed that virus productivity was also similar. Such results suggest that the conditions already selected for the production of the vaccinia virus in the STR can be hereinafter used because the above process conditions selected on a small scale of 30 mL EMF scale are suitable for the conditions for producing the vaccinia virus on the 1.8 L STR scale. Therefore, a scale-up from EMF to STR can be achieved.

### Example 4. Selection of culture medium for suspended HeLa S3 cells

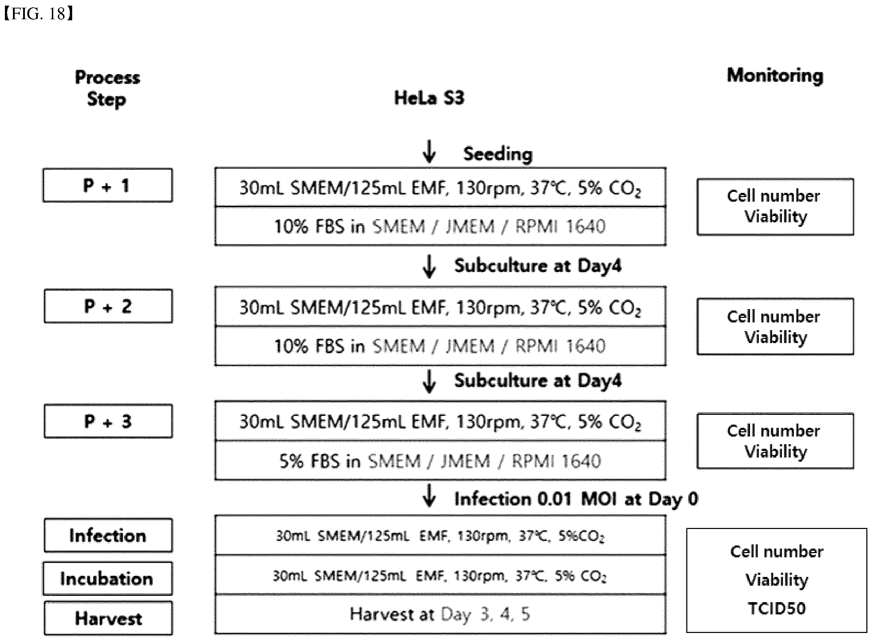

The present inventors performed an experiment for selecting a culture medium for HeLa S3 cells in order to further increase virus productivity or secure an effect of reducing production costs under the conditions selected through Examples 1 to 3. Initial cell culture and virus production were performed according to the conditions and procedure illustrated in FIG. 18, the cells were cultured by changing the culture medium for the cells to SMEM, JMEM, and RPMI1640, respectively, and virus productivity was confirmed by infecting the cells with the vaccinia virus at P+3. In addition, the experiment was performed by setting the harvest day at days 3 to 5 in anticipation of changes in virus productivity caused by the medium.

### 4-1. Analysis of cell growth according to medium type

First, the live cell number and cell viability were analyzed in order to investigate how the change in medium type affects the growth of the suspended HeLa S3 cells.

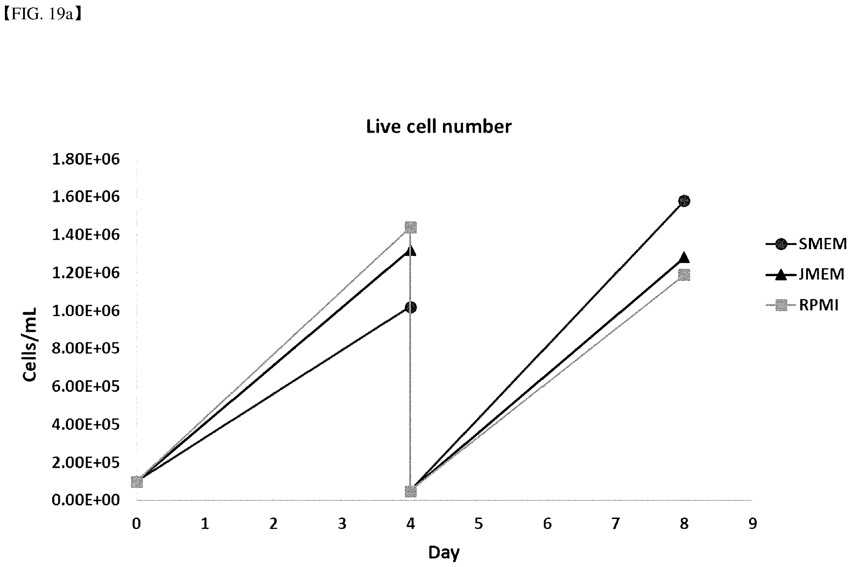

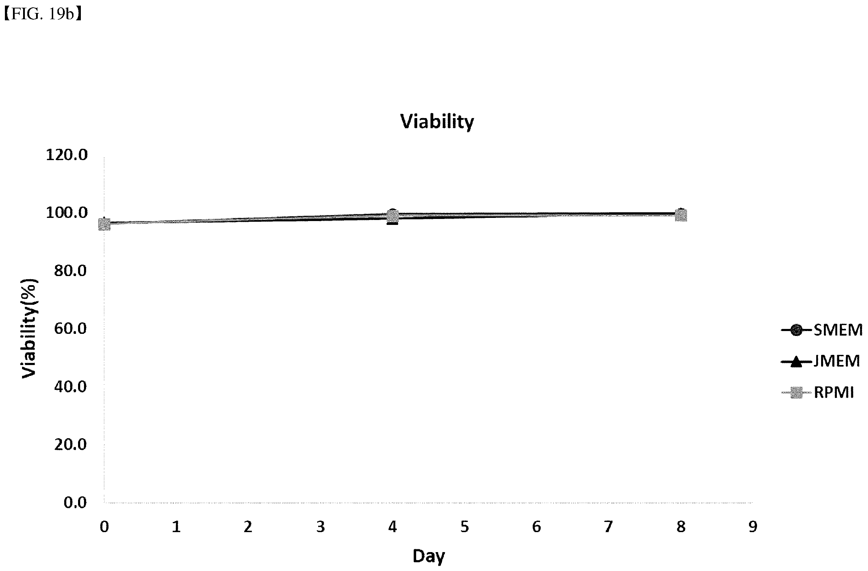

As a result, as illustrated in FIGS. 19A and 19B, there was no significant difference in cell number and viability when three media were used after 2 days of culture. Therefore, it was determined that changing the medium would not cause any major problems in culturing HeLa S3 cells, and apart from the productivity of the vaccinia virus, it was determined that the RPMI 1640 medium, which is easier to supply and cheaper, is advantageous in terms of actual virus production.

### 4-2. Analysis of virus productivity according to medium type

The present inventors observed that HeLa S3 cells were similarly cultured regardless of the type of medium, and based on these results, an experiment for investigating the effect of the type of medium on actual virus productivity was performed. For this purpose, after the cells were cultured, they were infected with the virus and virus productivity was comparatively analyzed through TCID50.

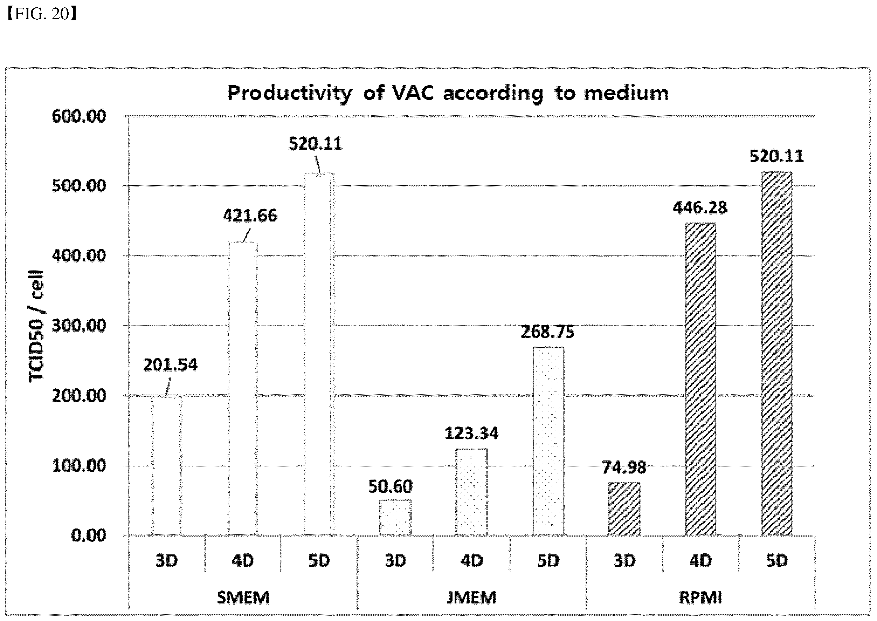

As a result, as illustrated in FIG. 20, it was confirmed that except for the JMEM medium, when SMEM and RPMI 1640, which had been used in the related art, were used, virus productivity was better than the internal standard of 300 TCID50/cell or more and was shown to be at a similar level. Through these results, it was concluded that RPMI 1640 may be used during the production of the vaccinia virus. Overall, in consideration of both the culture efficiency of the HeLa S3 cells and the productivity of the vaccinia virus, it was determined that it is appropriate to use the RPMI 1640 medium, which is easy to supply and can reduce costs, and accordingly, RPMI 1640 was finally selected as a medium for cell culture and virus production.

### 4-3. Derivation of final production process of vaccinia virus using suspended HeLa S3 cells

Through the results of the examples, final process conditions capable of efficiently producing vaccinia virus in suspended HeLa S3 cells were established. The final process conditions are shown in the following Table 6, and it could be seen that when the virus was produced under such conditions, it is possible to produce the virus at a level equal to or higher than 300 TCID50/cell, which is the vaccinia virus productivity in the adherent HeLa cell, which was used as an internal standard by using suspended cells.

**[Table 6]**

| **Virus** | | Vaccinia virus (IHD-W strain) |
|---|---|---|
| **Cell line** | | HeLa S3 |
| **Culture condition** | | RPMI-1640 (10% FBS, 37°C 5% CO₂) |
| **Virus Infection** | **Seeding density (seeding density)** | 5*10⁴ cells/mL |
| | **Infection time** | 16 hours after cell seeding |
| | **Infection condition** | 30 mL of RPMI-1640 supplemented with 5% FBS |
| | **MOI** | 0.01 MOI |
| | **Cell culture conditions after infection with virus** | RPMI-1640 supplemented with 5% FBS, 37°C 5% CO₂ |
| | **Culture period** | Day 5 |

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive.

### [Industrial Applicability]

The present invention relates to a method for mass-producing vaccinia virus using suspended cells, and in order to mass-produce the vaccinia virus, which was impossible to mass-produce in the related art, a technique of mass-producing the vaccinia virus was secured by specifically establishing the appropriate cell number, MOI, culture FBS concentration, and medium conditions. Accordingly, the technique of producing vaccinia virus using suspended cells according to the present invention enables mass production of vaccinia virus with high productivity. Since it is possible to reduce production costs and time, manpower, and the like using suspended cells, it is expected that the technique will be effectively used in clinical and commercial production fields that require mass production of vaccinia virus.

## Claims

1. A method for mass-producing vaccinia virus, the method comprising the following steps:
(a) initially culturing suspended HeLa S3 or Madin-Darby canine kidney (MDCK) cells;
(b) subculturing the initially cultured cells, seeding the subcultured cells at a density of 5.00E+04 to 1.00E+05 cells/mL, and then infecting the cells with vaccinia virus at a multiplicity of infection (MOI) of 0.01 to 0.1 TCID50/cell and culturing the infected cells; and
(c) harvesting the virus from the cell culture.

2. The method of claim 1, wherein the initial culturing in Step (a) is culturing the cells until passage 2 to 4.

3. The method of claim 2, wherein the initial culturing in Step (a) is culturing the cells until passage 2.

4. The method of claim 2 or 3, wherein in Step (a), cells in each passage are cultured for 3 to 5 days.

5. The method of claim 3, wherein in the initial culture, cells in passage 1 are seeded at a density of 1.00E+05 to 3.00E+05 cells/mL, and cells in passage 2 are seeded at a density of 5.00E+04 to 1.00E+05 cells/mL.

6. The method of claim 1, wherein the cells are cultured in a medium supplemented with fetal bovine serum (FBS).

7. The method of claim 6, wherein the medium is a serum-like modified Eagle's medium (SMEM) or a RPMI 1640 medium.

8. The method of claim 6, wherein the fetal bovine serum is added at a concentration of 5% to 10%.

9. The method of claim 1, wherein the harvesting in Step (c) is performed 4 to 6 days after infecting the cells with the virus.

10. The method of claim 1, wherein the vaccinia virus is any one strain selected from the group consisting of Western Reserve (WR), New York vaccinia virus (NYVAC), The New York City Board of Health (Wyeth), LC16m8, Lister, Copenhagen, Tian Tan, USSR, TashKent, Evans, International Health Division-J (IHD-J), International Health Division-White (IHD-W), variants thereof and combinations thereof.
